# EUROPÄISCHE PATENTSCHRIFT

(11) **EP 2 789 247 B1**
(45) Veröffentlichungstag und Bekanntmachung des Hinweises auf die Patenterteilung: **18.11.2015**
(21) Anmeldenummer: 13163647.4
(22) Anmeldetag: 14.04.2013
(51) Int. Cl.: A61K 8/36, A61K 36/736, A61K 31/352, A61K 31/192, A23L 1/30, A61K 8/49

(54) **ANTIOXIDATIVE ZUBEREITUNG**
ANTIOXIDATIVE COMPOSITION
COMPOSITION ANTIOXYDANTE

(43) Veröffentlichungstag der Anmeldung: 15.10.2014
(73) Patentinhaber: Symrise AG, 37603 Holzminden (DE)
(72) Erfinder: Stürtz, Melanie, 37671 Höxter (DE); Erfurt, Harry, 37170 Uslar (DE); Wintermeyer, Christian, 37671 Höxter (DE); Schuld, René, 22880 Wedel (DE); Niemeyer, Hans-Jürgen, 37603 Holzminden (DE)
(74) Vertreter: Fabry, Bernd

(56) Entgegenhaltungen:
- EP-A1- 2 550 872
- WO-A1-2005/070380
- WO-A1-2013/016257
- DE-A1-102011 052 708
- LI ET AL.: "Comparison of antioxidant capacity and phenolic compounds of berries, chokeberry and seabuckthorn", CENT. EUR. J. BIOL., Bd. 4, Nr. 4, 2009, Seiten 499-506, XP002713313,

## Beschreibung

### Gebiet der Erfindung

Die Erfindung befindet sich auf dem Gebiet der Antioxidantien und betrifft Zubereitungen, die eine synergistische Kombination von speziellen Hydroxyzimtsäuren, gegebenenfalls zusammen mit Polyphenolen enthalten, Extrakte von Pflanzen und Früchten, die diese enthalten, ein Herstellverfahren für entsprechende Extrakte speziell auf Basis von Sauerkirschen und Anwendungen der synergistischen Kombination von Antioxidantien in unterschiedlichsten Bereichen.

### Stand der Technik

Zu den Umwelteinflüssen, die eine besondere Gefahr für den menschlichen Organismus darstellen, zählen insbesondere oxidative Schädigungen von Zellen, speziell auch vom Erbgut. Energiereiche Strahlung insbesondere zusammen mit Sauerstoff erzeugen hochreaktive Radikale ("radical oxygen species"), die beispielsweise in der Lage sind, die DNA so zu verändern, dass Thymin zu Thyminglycol oder Guanin zu 8-Oxoguanin (8-OxoG) oxidiert wird. Gerade letzteres ist ein aggressives Mutagen, weil während der Replikation gegenüber einem 8-OxoG sowohl das normale Cytosin-Nucleotid, jedoch bevorzugt ein Adenin-Nucleotid eingebaut werden kann. Eine fehlerhafte Traversion gehört zu den Ursachen, für Zelldefekte und schließlich Krebs. Oxidativer Stress, ausgelöst durch ROS führt auch zu einer beschleunigten Hautalterung, was etwa bei übermäßiger Exposition von UV-Strahlen (beispielsweise in Bräunungsstudios) regelmäßig zu beobachten ist.

Es liegt daher auf der Hand, dass ein erhebliches Interesse daran besteht, den menschlichen Organismus vor derartigen oxidativen Schädigungen zu schützen. Dies kann beispielsweise durch die Verabreichung von Stoffen geschehen, die die Radikale abfangen ("quenchen"), wobei diese so genannten Antioxidantien oral aufgenommen werden können - entweder mit der Nahrung oder separat als Nahrungsmittelergänzungsstoffe- oder topische Anwendung finden, beispielsweise in kosmetische Produkten, die mit der Haut in Kontakt gebracht werden.

Als Antioxidantien haben sich unterschiedliche Naturstoffe durchgesetzt. Zu den bekanntesten zählen neben Vitamin C (Ascorbinsäure) die unterschiedlichen Carotinderivate (z.B. beta Carotin, Lycopin, Lutein) und insbesondere Vitamin A und seine Ableger (Tocopherol, Tocopherolacetat und Tocopherolpalmitat). Eine weitere wichtige Gruppe stellen die Polyphenole, speziell die Anthocyane und Isoflavone dar, die beispielsweise in einer Vielzahl von roten Früchten enthalten sind. Zwar ist bekannt, dass entsprechende Säfte und Extrakte dieser Früchte auch geringe Mengen an phenolischen Säuren und Hydroxyzimtsäuren, insbesondere Chlorogensäure enthalten, im Hinblick auf ihren Einfluss auf das antioxidative Potential dieser anthocyanreichen Extrakte ist diesen Begleitstoffen bislang aber wenig Beachtung geschenkt worden.

DE 10 2011 052 708 beschreibt ein Verfahren zur Stabilisierung von photolabilen Wirkstoffen in Lebens- und Futtermitteln durch Polyphenole dadurch gekennzeichnet, daß es sich bei den Polyphenolen um Benzoesäurederivate (beispielsweise Vanillinsäure, Gallussäure und Protocatechusäure), Zimtsäurederivate (beispielsweise Kaffeesäure, p-Cumarsäure, Sinapinsäure, Fumarsäure) oder Quercetin handelt.

Nun besteht im Markt ein beständiges Interesse daran, das Leistungsvermögen und das Leistungsspektrum solcher natürlichen Antioxidantien zu verbessern, sei es, dass mit gleichen Mengen eine höhere Leistung erzielt werden soll oder umgekehrt die gleiche Leistung mit geringeren Mengen angestrebt wird.

Die Aufgabe der vorliegenden Erfindung hat somit darin bestanden, bestehende Polyphenolhaltige Zubereitungen, speziell Extrakte mit einem hohen Gehalt an Anthocyanen bzw. Anthocyanidinen in Richtung einer erhöhten antioxidativen Leistung zu verbessern.

### Beschreibung der Erfindung

In einer ersten Ausführungsform betrifft der Gegenstand der Erfindung eine antioxidative Zubereitung, enthaltend
(a) Kaffeesäure,
(b) Sinapinsäure sowie gegebenenfalls
(c) Polyphenole,
mit der Maßgabe, dass die Komponenten (a) und (b) im Gewichtsverhältnis von etwa 85:15 bis 98:2, und insbesondere etwa 87:13 bis 97:3 zugegen sind. Vorzugsweise sind die sie die Komponenten (a+b) und (c) im Gewichtsverhältnis von etwa 5:95 bis etwa 1:99 und vorzugsweise etwa 4:96 bis etwa 2:98 enthalten.

In einer zweiten Ausführungsform wird ein Extrakt beansprucht, der
(a) Kaffeesäure,
(b) Sinapinsäure sowie gegebenenfalls
(c) Polyphenole,
mit der Maßgabe enthält, dass die Komponenten (a) und (b) im Gewichtsverhältnis von etwa 85:15 bis 98:2, vorzugsweise etwa 87:13 bis 97:3 zugegen sind.

Überraschenderweise wurde gefunden, dass unter den in Anthocyanextrakten enthaltenen Hydroxyzimtsäuren gerade Kaffeesäure und Sinapinsäure bezüglich ihrer antioxidativen Eigenschaften eine starke Synergie zeigen, wie dies im ORAC Test nachgewiesen werden konnte. Diese positiven Eigenschaften sind zudem schon bereits in sehr kleine Einsatzmengen der beiden Stoffe nachweisbar und verstärken sich im Zusammenspiel mit Polyphenolen, insbesondere Anthocyanen.

Die Zubereitungen können sowohl als solche eingesetzt werden, als auch in Form von Extrakten, die aus Pflanzen oder Früchten nach einem speziellen Verfahren erhältlich sind. Sowohl die Zubereitungen als auch die Extrakte eignen sich in vorzüglicher Weise als Zusatzstoffe für Nahrungsmittel, Nahrungsergänzungsmittel, aber auch zur Herstellung von pharmazeutischen Zubereitungen, die auf eine Verbesserung des Schutzes von Zellen gegen oxidative Schädigungen beispielsweise durch UV-Strahlung abzielen. Neben diesen Anwendungen, bei denen die Produkte oral aufgenommen werden, kommt auch eine topische Anwen-dung in Betracht, wobei die Zubereitungen oder Extrakte in kosmetische Zubereitungen, speziell in Haut-, Haar- und Sonnenschutzprodukte eingearbeitet werden.

### Hydroxyzimtsäuren

### Kaffeesäure (3,4-Dihydroxyzimtsäure) und Sinapinsäure (3-(4-Hydroxy-3,5-dimethoxy-phenyl)-2-propensäure)

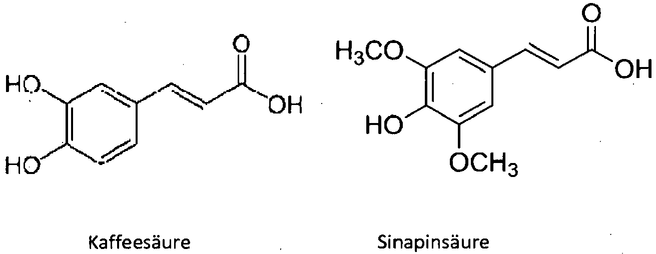

gehören zu den Hydroxyzimtsäuren, die in vielen Pflanzen und Früchten, speziell in Beeren enthalten sind.

### Polyphenole

Bei den Polyphenole, die die fakultative Komponente (c) bilden, handelt es sich vorzugsweise um Anthocyane bzw. Anthocyanidine.

***Anthocyadine*** sind chymochrome Farbstoffe die die allgemeine Struktur (I) aufweisen, 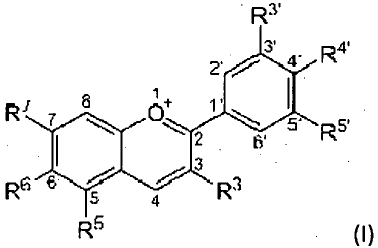 die nur im Zellsaft von Landpflanzen, nicht aber in Tieren, Mikroorganismen oder Wasserpflanzen zu finden sind. Anthocyane kommen nahezu in allen höheren Pflanzen, meist in den Blüten und Früchten, aber auch in den Blättern und Wurzeln vor. In den jeweiligen Pflanzenteilen sind sie vor allem in den äußeren Zellschichten wie den Epidermiszellen zu finden. Die dort gefundenen Mengen sind relativ groß: Ein Kilogramm Brombeeren enthält zum Beispiel etwa 1,15 Gramm Anthocyane, aus roten und schwarzen Hülsenfrüchten lassen sich bis zu 20 Gramm pro Kilogramm Schale gewinnen. Tabelle A gibt einen Überblick über die wichtigsten Anthocyane:

**Table A**

| Anthocyane | | | | | | | | |
|---|---|---|---|---|---|---|---|---|
| **Anthocyanidine** | **Basisstruktur** | **R_{3'}** | **R_{4'}** | **R_{5'}** | **R₃** | **R₅** | **R₆** | **R₇** |
| Aurantinidin | 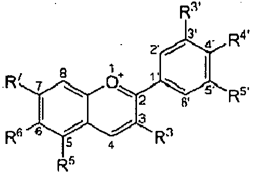 | -H | -OH | -H | -OH | -OH | -OH | -OH |
| Cyanidin | | -OH | -OH | -H | -OH | -OH | -H | -OH |
| Delphinidin | | -OH | -OH | -OH | -OH | -OH | -H | -OH |
| Europinidin | | -OCH₃ | -OH | -OH | -OH | -OCH₃ | -H | -OH |
| Luteolinidin | | -OH | -OH | -H | -H | -OH | -H | -OH |
| Pelargonidin | | -H | -OH | -H | -OH | -OH | -H | -OH |
| Malvidin | | -OCH₃ | -OH | -OCH₃ | -OH | -OH | -H | -OH |
| Peonidin | | -OCH₃ | -OH | -H | -OH | -OH | -H | -OH |
| Petunidin | | -OH | -OH | -OCH₃ | -OH | -OH | -H | -OH |
| Rosinidin | | -OCH₃ | -OH | -H | -OH | -OH | -H | -OCH₃ |

Am häufigsten kommen in der Natur die Glykoside von Cyanidin, Delphinidin, Malvidin, Pelargonidin, Peonidin und Petunidin vor, die als ***Anthocyane*** bezeichnet werden. Im Sinne der Erfindung sind Glukoside, Rutinsoide, Rhamnoside, Galactosid und Arabinoside von Cyanidin, Delphinidin, Peonidin und Petunidin, wie sie in besonders hohen Konzentrationen beispielsweise in der Sauerkirsche enthalten sind, besonders bevorzugt. Dem entsprechend bevorzugt sind daher auch entsprechende Extrakte.

Neben den Anthocyanen bzw. Anthocyanidinen können die Zubereitungen bzw. Extrakte vorteilhafterweise jedoch auch Flavone oder Flavonglykoside, wie beispielsweise Catechine (z.B. Catechin, Epicatechin, Epigallocatechingallat (EGCG), Gallocatechin, Gallocatechin-3-gallat, Epicatechingallat), Isocatechin oder Prunetin enthalten.

### Extrakte

Die erfindungsgemäßen Extrakte zeichnen sich vorzugsweise dadurch aus, dass sie
(a) etwa 150 bis 1.000 ppm, vorzugsweise etwa 300 bis 900 ppm und insbesondere etwa 550 bis 750 ppm Kaffeesäure,
(b) 10 bis 50 ppm, vorzugsweise etwa 12 bis 40 ppm und insbesondere etwa 15 bis 25 ppm Sinapinsäure, und
(c) 10 bis 60 Gew.-%, vorzugsweise etwa 20 bis 50 Gew.-% und insbesondere etwa 25 bis 45 Gew.-% Polyphenole sowie
ad 100 Gew.-% im Wesentlichen Lösungsmittel enthalten, wobei wieder die Auswahlregel gilt, nach der die Komponenten (a) und (b) im Gewichtsverhältnis von etwa 85:15 bis 98:2, vorzugsweise etwa 87:13 bis 97:3 zugegen sind. Darüber hinaus weisen die Extrakte einen Gesamtgehalt an Hydroxyzimtsäuren von etwa 0,5 bis etwa 3 Gew.-%, vorzugsweise 0,7 bis 1,5 auf. Weitere Bestandteile sind beispielsweise Zucker in untergeordneten Mengen.

Die Extrakte, speziell die Extrakte auf Basis von Sauerkirschen, zeichnen sich weiterhin dadurch aus, dass sie folgende Parameter mit der Maßgabe aufweisen, dass die Größen (a) bis (e) alle oder einzeln oder in beliebiger Kombination erfüllt sein können und daher alle möglichen Iterationen - auch bezüglich der Kombination von bevorzugten Bereichsangaben innerhalb verschiedener Parameter - hiermit ausdrücklich offenbart sind:
(a) einen ORAC-Wert von größer 5.000, vorzugsweise größer 6.000 und insbesondere größer 10.000; und/oder
(b) einen Polyphenolgehalt von größer 40 Gew.-%, vorzugsweise größer 45 Gew.-% und insbesondere größer 50 Gew.-%; und/oder
(c) einen Anthocyangehalt von etwa 6 bis etwa 25 Gew.-%, vorzugsweise etwa 15 bis etwa 20 Gew.-% und insbesondere etwa 16 bis etwa 18 Gew.-%; und/oder
(d) einen Aschegehalt von kleiner 3 Gew.-%, vorzugsweise kleiner 2 Gew.-% und insbesondere etwa 0,5 bis etwa 1 Gew.-%; und/oder
(e) einen Zuckergehalt von kleiner 0,25 Gew.-%, vorzugsweise kleiner 0,1 Gew.-% und insbesondere von 0 Gew.-%.

Die Extrakte werden auf Basis Pflanzen oder Früchten gewonnen, die ausgewählt sind aus der Gruppe, die gebildet wird
- **Açai** (*Euterpe oleracea)*;
- **Aronia** (e.g. *Aronia arbutifolia, Aronia melanocarpa; Aronia prunifolia*)*;*
- **Schwarze und Rote Johannisbeeren** (e.g. *Ribes rubrum, Ribes spciatum, Ribes alpinum, Ribes schlechtendalii, Ribes multiflorum, Ribes petraeum, Ribes trite, Ribes nigrum);*
- **Brombeeren** (*Rubus sp*.);
- **Schwarze Karotten** (*Daucus ccrrota*);
- **Schwarze Tomaten** (*Solanum lycopersicum*);
- **Blutorangen** (*Citrus sinensis*);
- **Blaubeeren** (*Vaccinium corymbosum, Vaccinium angustifolium*);
- **Schwarzdorn** (*Prunus spinosa*);
- **Rauschbeeren** (*Vaccinium ulginosum*);
- **Moltebeeren** (*Rubus chamaemorus*);
- **Preiselbeeren** (e.g. *Vaccinium oxycoccos, Vaccinium microcarpus, Vaccinium macrocarpus, Vaccinium erythrocarpus*);
- **Krähenbeeren** (e.g. *Empetrum nigrum, Empetrum eamesii, Empetrum rumbrum, Empetrum hermaphroditum*);
- **Holunderbeeren** (e.g. *Sambucus nigra, Sambucus racemosa*);
- **Hibiskus** *(Hibiscus sabdariffa, Roselle*);
- **Kronsbeeren** (e.g. *Vaccinium vitus idaea*);
- **Magellanbeeren** ("Calafate", *Berberis microphylla, Breberis buxifolia);*
- **Makibeeren** (e.g. *Aristotelia chilensis*)*;*
- **Bergbeeren** (*Vaccinium membranaceum*);
- **Pflaumen und Zwetschgen** (*Prunus domestica*)*;*
- **Himbeeren** (*Rubus idaeus, Rubus occidentalis*)*;*
- **Rote Stachelbeeren** (*Ribes uva-crispa, Ribes grossularia*)*;*
- **Rote Trauben** (*Vitis vinifera, Vitis labrusca, Vitis riparia, Vitis rotundifolia*)*;*
- **Rowanbeeren** (*Sorbus aucuparia*);
- **Sauerkirschen** (e.g. *Prunus avium, Prunus cerasus*)*;*
- **Erdbeeren** (*Fragaria ananassa*)*;*
- **Süßkirschen** (*Prunus avium*)*;*
- **Taybeeren** (*Rubus X*).

Es ist des Weiteren vorteilhaft, wenn der Gehalt an Disacchariden in den Zusammensetzungen weniger als 2 Gew.-%, insbesondere etwa 0,1 bis 1,5 Gew.-% beträgt. Es ist ferner typisch, dass bevorzugte Zubereitungen einen signifikanten Kaliumgehalt von insbesondere etwa 10 bis etwa 5.000, vorzugsweise etwa 100 bis etwa 2.500 und besonders bevorzugt etwa 200 bis etwa 1.000 ppm aufweist.

Ein weiterer Gegenstand der Erfindung betrifft ein Verfahren zur Herstellung eines entsprechenden Extraktes, welcher Kaffee- und Sinapinsäure im synergistischen Gewichtsverhältnis enthält, bei dem man
(i) aus den Pflanzen oder Früchten einen Saft oder Auszug herstellt und ansäuert,
(ii) das angesäuerte Produkt über eine Adsorbersäule gibt, die als Adsorptionsmittel ein makroporöses vernetztes nicht-funktionalisiertes Polystyrolharz enthält,
(iii) die Mischung enthaltend die Komponenten (a), (b) und (c) mit wässrigem, bevorzugt angesäuertem Alkohol desorbiert und schließlich
(iv) das Eluat trocknet.

Der Saft kann aus den Früchten vorzugsweise durch Pressen hergestellt werden. Werden beispielsweise Hibiskusblätter eingesetzt, kann man alternativ einen Auszug einsetzten, den man durch Extraktion oder Wasserdampfdestillation gewinnt. Dem entsprechend kann der Auszug wässrig, alkoholisch oder wässrig-alkoholisch sein. Je nach Konzentration empfiehlt es sich den Saft oder Auszug mit Wasser zu verdünnen, beispielsweise so weit, dass die Menge an nicht-wässrigen Bestandteilen etwa 15 bis 35, vorzugsweise etwa 20 bis 25 Gew.-% beträgt. Um die Anthocyane und Hydroxyzimtsäuren stabil in Lösung zu halten, empfiehlt es sich, die Verdünnungen beispielsweise mit Ameisensäure, Milchsäure oder Zitronensäure anzusäuern und auf einen pH-Wert im Bereich von etwa 2 bis 4, vorzugsweise etwa 3 einzustellen. Anschließend wird die angesäuerte Verdünnung auf eine Adsorbersäule gegeben, die ein Harz enthält, welches selektiv für die Hydroxyzimtsäuren und Polyphenole ist. Es hat sich erwiesen, dass für diesen Zweck makroporöse vernetzte Polystyrolharze geeignet sind, die keine weiteren funktionellen Gruppen tragen. Diese sind nämlich nicht nur spezifisch für die Anthocyane bzw. Anthocyanidine, sondern reichern überraschenderweise die beiden Hydroxyzimtsäuren (a) und (b) im synergistischen Verhältnis an. Typische Beispiele für geeignete Adsorberharze umfassen das Produkt Lewatit^{®} VP OC 1064 MD PH, Amberlite XAD7 (Lanxess) oder Symtrap^{®} (Symrise). Die Desorption erfolgt dann mit wässrigem Alkohol, speziell wässrigem Ethanol.

### Nahrungs- und Nahrungsergänzungsmittel

Ein weiterer Gegenstand der Erfindung betrifft Nahrungs- oder Nahrungsergänzungsmittel, enthaltend entweder antioxidative Zubereitungen, enthaltend
(a) Kaffeesäure,
(b) Sinapinsäure sowie gegebenenfalls
(c) Polyphenole,
oder
Extrakte enthaltend
(a) Kaffeesäure,
(b) Sinapinsäure sowie gegebenenfalls
(c) Polyphenole,
jeweils mit der Maßgabe, dass die Komponenten (a) und (b) im Gewichtsverhältnis von etwa 85:15 bis 98:2, vorzugsweise etwa 87:13 bis 97:3 zugegen sind.

Soweit die konfektionierten Produkte Nahrungsmittel darstellen, denen man die antioxidativen Zubereitungen oder die Extrakte direkt zusetzt, handelt es sich beispielsweise um Backwaren, beispielsweise Brot, Trockenkekse, Kuchen, sonstiges Gebäck, Süßwaren (beispielsweise Schokoladen, Schokoladenriegelprodukte, sonstige Riegelprodukte, Fruchtgummi, Hart- und Weichkaramellen, Kaugummi), alkoholische oder nicht-alkoholische Getränke (beispielsweise Kaffee, Tee, Eistee, Wein, weinhaltige Getränke, Bier, bierhaltige Getränke, Liköre, Schnäpse, Weinbrände, (carbonisierte) fruchthaltige Limonaden, (carbonisierte) isotonische Getränke, (carbonisierte) Erfrischungsgetränke, Nektare, Schorlen, Obst- und Gemüsesäfte, Frucht- oder Gemüsesaftzubereitungen, Instantgetränke (beispielsweise Instant-Kakao-Getränke, Instant-Tee-Getränke, Instant-Kaffeegetränke, Instant-Fruchtgetränke), Fleischprodukte (beispielsweise Schinken, Frischwurst- oder Rohwurstzubereitungen, gewürzte oder marinierte Frisch- oder Pökelfleischprodukte), Eier oder Eiprodukte (Trockenei, Eiweiß, Eigelb), Getreideprodukte (beispielsweise Frühstückscerealien, Müsliriegel, vorgegarte Fertigreis-Produkte), Milchprodukte (beispielsweise Milchgetränke, Buttermilchgetränke, Milcheis, Joghurt, Kefir, Frischkäse, Weichkäse, Hartkäse, Trockenmilchpulver, Molke, Molkegetränke, Butter, Buttermilch, teilweise oder ganz hydrolisierte Milchproteinhaltige Produkte), Produkte aus Sojaprotein oder anderen Sojabohnen-Fraktionen (beispielsweise Sojamilch und daraus gefertigte Produkte, Fruchtgetränke mit Sojaprotein, Sojalecithin-haltige Zubereitungen, fermentierte Produkte wie Tofu oder Tempe oder daraus gefertigte Produkte), Produkte aus anderen pflanzlichen Proteinquellen, beispielsweise Haferprotein-Getränke, Fruchtzubereitungen (beispielsweise Konfitüren, Fruchteis, Fruchtsoßen, Fruchtfüllungen), Gemüsezubereitungen (beispielsweise Ketchup, Soßen, Trockengemüse, Tiefkühlgemüse, vorgegarte Gemüse, eingekochte Gemüse), Knabberartikel (beispielsweise gebackene oder frittierte Kartoffelchips oder Kartoffelteigprodukte, Extrudate auf Mais- oder Erdnussbasis), Produkte auf Fett- und Ölbasis oder Emulsionen derselben (beispielsweise Mayonnaise, Remoulade, Dressings), sonstige Fertiggerichte und Suppen (beispielsweise Trockensuppen, Instant-Suppen, vorgegarte Suppen), Gewürze, Würzmischungen sowie insbesondere Aufstreuwürzungen (englisch: Seasonings), die beispielsweise im Snackbereich Anwendung finden. Die antioxidativen Zubereitungen oder die Extrakte können insbesondere in Sportgetränken eingesetzt werden, darunter insbesondere in solchen Sportgetränken, die der Regeneration des Sportlers nach einer intensiven sportlichen Tätigkeit dienen oder die die Leistungsfähigkeit steigern.

Die Zubereitungen werden üblicherweise in Mengen von etwa 0,1 bis 5, vorzugsweise etwa 0,5 bis 3 und insbesondere etwa 1 bis 2 Gew.-% zugesetzt. Für die Extrakte gelten die gleichen Mengenangaben; gegebenenfalls beziehen sie sich auf den Wirkstoffgehalt.

### 1. Kapseln

Handelt es sich bei den Produkten um Nahrungsmittelergänzungsstoffe, so werden diese in der Regel ohne weitere Zusatzstoffe eingesetzt, wobei von reinen Konfektionierungsmittel abzusehen ist. Eine bevorzugte Anwendungsform stellen hier Makro- oder Mikrokapseln dar. Makrokapseln bestehen vorzugsweise aus Gelatine oder es handelt sich um sprühgetrocknete Produkte, die als Basis Polysaccharide oder Dextrine enthalten. Diese weisen in der Regel Teilchendurchmesser von 0,5 bis 1,5 cm auf.

Unter den Begriffen "Mikrokapsel" oder "Nanokapsel" werden vom Fachmann hingegen sphärische Aggregate mit einem Durchmesser im Bereich von etwa 0,0001 bis etwa 5 und vorzugsweise 0,005 bis 0,5 mm verstanden, die mindestens einen festen oder flüssigen Kern enthalten, der von mindestens einer kontinuierlichen Hülle umschlossen ist. Genauer gesagt handelt es sich um mit filmbildenden Polymeren umhüllte feindisperse flüssige oder feste Phasen, bei deren Herstellung sich die Polymere nach Emulgierung und Koazervation oder Grenzflächenpolymerisation auf dem einzuhüllenden Material niederschlagen. Nach einem anderen Verfahren werden geschmolzene Wachse in einer Matrix aufgenommen ("microsponge"), die als Mikropartikel zusätzlich mit filmbildenden Polymeren umhüllt sein können. Nach einem dritten Verfahren werden Partikel abwechselnd mit Polyelektrolyten unterschiedlicher Ladung beschichtet ("layer-by-layer"-Verfahren). Die mikroskopisch kleinen Kapseln lassen sich wie Pulver trocknen. Neben einkernigen Mikrokapseln sind auch mehrkernige Aggregate, auch Mikrosphären genannt, bekannt, die zwei oder mehr Kerne im kontinuierlichen Hüllmaterial verteilt enthalten. Ein- oder mehrkernige Mikrokapseln können zudem von einer zusätzlichen zweiten, dritten etc. Hülle umschlossen sein. Die Hülle kann aus natürlichen, halbsynthetischen oder synthetischen Materialien bestehen. Natürlich Hüllmaterialien sind beispielsweise Gummi Arabicum, Agar-Agar, Agarose, Maltodextrine, Alginsäure bzw. ihre Salze, z.B. Natrium- oder Calciumalginat, Fette und Fettsäuren, Cetylalkohol, Collagen, Chitosan, Lecithine, Gelatine, Albumin, Schellack, Polysaccharide, wie Stärke oder Dextran, Polypeptide, Proteinhydrolysate, Sucrose und Wachse. Halbsynthetische Hüllmaterialien sind unter anderem chemisch modifizierte Cellulosen, insbesondere Celluloseester und -ether, z.B. Celluloseacetat, Ethylcellulose, Hydroxypropylcellulose, Hydroxypropylmethylcellulose und Carboxymethylcellulose, sowie Stärkederivate, insbesondere Stärkeether und -ester. Synthetische Hüllmaterialien sind beispielsweise Polymere wie Polyacrylate, Polyamide, Polyvinylalkohol oder Polyvinylpyrrolidon. Beispiele für Mikrokapseln des Stands der Technik sind folgende Handelsprodukte (in Klammern angegeben ist jeweils das Hüllmaterial) : *Hallcrest Microcapsules* (Gelatine, Gummi Arabicum), *Coletica Thalaspheres* (maritimes Collagen), *Lipotec Millicapseln* (Alginsäure, Agar-Agar), *Induchem Unispheres* (Lactose, mikrokristalline Cellulose, Hydroxypropylmethylcellulose); *Unicerin C30* (Lactose, mikrokristalline Cellulose, Hydroxypropylmethylcellulose), *Kobo Glycospheres* (modifizierte Stärke, Fettsäureester, Phospholipide), *Softspheres* (modifiziertes Agar-Agar) und *Kuhs Probiol Nanospheres* (Phospholipide) sowie *Primaspheres* und *Primasponges* (Chitosan, Alginate) und *Primasys* (Phospholipide).

### 2. Kaugummis

Eine weitere Darreichungsform der anti-oxidativen Zubereitungen kann in Form von Kaugummis erfolgen. Diese Produkte enthalten typischerweise eine wasserunlösliche und eine wasserlösliche Komponente.

Die wasserunlösliche Basis, die auch als "Gummibasis" bezeichnet wird. umfasst üblicherweise natürliche oder synthetische Elastomere, Harze, Fette und Öle, Weichmacher, Füllstoffe, Farbstoffe sowie gegebenenfalls Wachse. Der Anteil der Basis an der Gesamtzusammensetzung macht üblicherweise 5 bis 95, vorzugsweise 10 bis 50 und insbesondere 20 bis 35 Gew.-% aus. In einer typischen Ausgestaltungsform der Erfindung setzt sich die Basis aus 20 bis 60 Gew.-% synthetischen Elastomeren, 0 bis 30 Gew.-% natürlichen Elastomeren, 5 bis 55 Gew.-% Weichmachern, 4 bis 35 Gew.-% Füllstoffe und in untergeordneten Mengen Zusatzstoffe wie Farbstoffe, Antioxidantien und dergleichen zusammen, mit der Maßgabe, dass sie allenfalls in geringen Mengen wasserlöslich sind.

Als geeignete synthetische Elastomere kommen beispielsweise Polyisobutylene mit durchschnittlichen Molekulargewichten (nach GPC) von 10.000 bis 100.000 und vorzugsweise 50.000 bis 80.000, Isobutylen-Isopren-Copolymere ("Butyl Elastomere"), Styrol-Butadien-Copolymere (Styrol:Butadien-Verhältnis z.B. 1: 3 bis 3: 1), Polyvinylacetate mit durchschnittlichen Molekulargewichten (nach GPC) von 2.000 bis 90.000 und vorzugsweise 10.000 bis 65.000, Polyisoprene, Polyethylen, Vinylacetat-Vinyllaurat-Copolymere und deren Gemische. Beispiele für geeignete natürliche Elastomere sind Kautschuks wie etwa geräucherter oder flüssiger Latex oder Guayule sowie natürliche Gummistoffe wie Jelutong, Lechi caspi, Perillo, Sorva, Massaranduba balata, Massaranduba chocolate, Nispero, Rosindinba, Chicle, Gutta hang 1kang sowie deren Gemische. Die Auswahl der synthetischen und natürlichen Elastomere und deren Mischungsverhältnisse richtet sich im Wesentlichen danach, ob mit den Kaugummis Blasen erzeugt werden sollen ("bubble gums") oder nicht. Vorzugsweise werden Elastomergemische eingesetzt, die Jelutong, Chicle, Sorva und Massaranduba enthalten.

In den meisten Fällen erweisen sich die Elastomere in der Verarbeitung als zu hart oder zu wenig verformbar, so dass es sich als vorteilhaft erwiesen hat, spezielle Weichmacher mitzuverwenden, die natürlich insbesondere auch alle Anforderungen an die Zulassung als Nahrungsmittelzusatzstoffe erfüllen müssen. In dieser Hinsicht kommen vor allem Ester von Harzsäuren in Betracht, beispielsweise Ester von niederen aliphatischen Alkoholen oder Polyolen mit ganz oder teilweise gehärteten, monomeren oder oligomeren Harzsäuren. Insbesondere werden für diesen Zweck die Methyl-, Glycerin-, oder Pentareythritester sowie deren Gemische eingesetzt. Alternativ kommen auch Terpenharze in Betracht, die sich von alpha-Pinen, beta-Pinen, delta-Limonen oder deren Gemischen ableiten können.

Als Füllstoffe oder Texturiermittel kommen Magnesium- oder Calciumcarbonat, gemahlener Bimsstein, Silicate, speziell Magnesium- oder Aluminiumsilicate, Tone, Aluminiumoxide. Talkum, Titandioxid, Mono-, Di- und Tricalciumphosphat sowie Cellulosepolymere.

Geeignete Emulgatoren sind Talg, gehärteter Talg, gehärtete oder teilweise gehärtete pflanzliche Öle, Kakaobutter, Partialglyceride, Lecithin, Triacetin und gesättigte oder ungesättigte Fettsäuren mit 6 bis 22 und vorzugsweise 12 bis 18 Kohlenstoffatomen sowie deren Gemische.

Als Farbstoffe und Weißungsmittel kommen beispielsweise die für die Färbung von Lebensmitteln zugelassenen FD und C-Typen, Pflanzen- und Fruchtextrakte sowie Titandioxid in Frage.

Die Basismassen können Wachse enthalten oder wachsfrei sein; Beispiele für wachsfreie Zusammensetzungen finden sich unter anderem in der Patentschrift US 5,286,500, auf deren Inhalt hiermit ausdrücklich Bezug genommen wird.

Zusätzlich zu der wasserunlöslichen Gummibasis enthalten Kaugummizubereitungen regelmäßig einen wasserlösliche Anteil, der beispielsweise von Softener, Süßstoffen, Füllstoffen, Geschmacksstoffen, Geschmacksverstärkern, Emulgatoren, Farbstoffen, Säuerungsmitteln, Antioxidantien und dergleichen gebildet werden, hier mit der Maßgabe, dass die Bestandteile eine wenigstens hinreichende Wasserlöslichkeit besitzen. In Abhängigkeit der Wasserlöslichkeit der speziellen Vertreter können demnach einzelne Bestandteile sowohl der wasserunlöslichen wie auch der wasserlöslichen Phase angehören. Es ist jedoch auch möglich, Kombinationen beispielsweise eines wasserlöslichen und eines wasserunlöslichen Emulgators einzusetzen, wobei sich die einzelnen Vertreter, dann in unterschiedlichen Phasen befinden. Üblicherweise macht der wasserunlösliche Anteil 5 bis 95 und vorzugsweise 20 bis 80 Gew.-% der Zubereitung aus.

Wasserlösliche Softener oder Plastifiziermittel werden den Kaugummizusammensetzungen hinzugegeben um die Kaubarkeit und das Kaugefühl zu verbessern und sind in den Mischungen typischerweise in Mengen von 0,5 bis 15 Gew.-% zugegen. Typische Beispiele sind Glycerin, Lecithin sowie wässrige Lösungen von Sorbitol, gehärteten Stärkehydrolysaten oder Kornsirup.

Als Süßstoffe kommen sowohl zuckerhaltige wie zuckerfreie Verbindungen in Frage, die in Mengen von 5 bis 95, vorzugsweise 20 bis 80 und insbesondere 30 bis 60 Gew.-% bezogen auf die Kaugummizusammensetzung eingesetzt werden. Typische Saccharid-Süssstoffe sind Sucrose, Dextrose, Maltose, Dextrin, getrockneter Invertzucker, Fructose, Levulose, Galactose, Kornsirup sowie deren Gemische. Als Zuckerersatzstoffe kommen Sorbitol, Mannitol, Xylitol, gehärtete Stärkehydrolysate, Maltitol und deren Gemische in Frage. Weiterhin kommen als Zusatzstoffe auch sogenannte HIAS ("High Intensity Articifical Sweeteners") in Betracht, wie beispielsweise Sucralose, Aspartam, Acesulfamsalze, Alitam, Saccharin und Saccharinsalze, Cyclamsäure und deren Salze, Glycyrrhizine, Dihydrochalcone, Thaumatin, Monellin und dergleichen alleine oder in Abmischungen. Besonders wirksam sind auch die hydrophoben HIAS, die Gegenstand der internationalen Patentanmeldung WO 2002 091849 A1 (Wrigleys) sowie Stevia Extrakte und deren aktiven Bestandteile, insbesondere Ribeaudiosid A sind. Die Einsatzmenge dieser Stoffe hängt in erster Linie von ihrem Leistungsvermögen ab und liegt typischerweise im Bereich von 0,02 bis 8 Gew.-%.

Insbesondere für die Herstellung kalorienarmer Kaugummis eignen sich Füllstoffe wie beispielsweise Polydextröse, Raftilose, Rafitilin, Fructooligosaccharide (NutraFlora), Palatinoseoligosaaccharide, Guar Gum Hydrolysate (Sun Fiber) sowie Dextrine.

Die Auswahl an weiteren Geschmacksstoffen ist praktisch unbegrenzt und für das Wesen der Erfindung unkritisch. Üblicherweise liegt der Gesamtanteil aller Geschmacksstoffe bei 0,1 bis 15 und vorzugsweise 0,2 bis 5 Gew.-% bezogen auf die Kaugummizusammensetzung. Geeignete weitere Geschmacksstoffe stellen beispielsweise essentielle Öle, synthetische Aromen und dergleichen dar, wie etwa Anisöl, Sternanisöl, Kümmelöl, Eukalyptusöl, Fenchelöl, Citronenöl, Wintergrünöl, Nelkenöl, und dergleichen, wie sie auch beispielsweise in Mund- und Zahnpflegemittel Verwendung finden.

Die Kaugummis können des weiteren Hilfs- und Zusatzstoffe enthalten, die beispielsweise für die Zahnpflege, speziell zur Bekämpfung von Plaque und Gingivitis geeignet sind, wie z.B. Chlorhexidin, CPC oder Trichlosan. Weiter können pH-Regulatoren (z.B. Puffer oder Harnstoff), Wirkstoffe gegen Karies (z.B. Phosphate oder Fluoride), biogene Wirkstoffe (Antikörper, Enzyme, Koffein, Pflanzenextrakte) enthalten sein, solange diese Stoffe für Nahrungsmittel zugelassen sind und nicht in unerwünschter Weise miteinander in Wechselwirkung treten.

Bei den bevorzugten oralen Zubereitungen kann es sich auch um Kaugummis handeln. Diese Produkte enthalten typischerweise eine wasserunlösliche und eine wasserlösliche Komponente.

Die wasserunlösliche Basis, die auch als "Gummibasis" bezeichnet wird. umfasst üblicherweise natürliche oder synthetische Elastomere, Harze, Fette und Öle, Weichmacher, Füllstoffe, Farbstoffe sowie gegebenenfalls Wachse. Der Anteil der Basis an der Gesamtzusammensetzung macht üblicherweise 5 bis 95, vorzugsweise 10 bis 50 und insbesondere 20 bis 35 Gew.-% aus. In einer typischen Ausgestaltungsform der Erfindung setzt sich die Basis aus 20 bis 60 Gew.-% synthetischen Elastomeren, 0 bis 30 Gew.-% natürlichen Elastomeren, 5 bis 55 Gew.-% Weichmachern, 4 bis 35 Gew.-% Füllstoffe und in untergeordneten Mengen Zusatzstoffe wie Farbstoffe, Antioxidantien und dergleichen zusammen, mit der Maßgabe, dass sie allenfalls in geringen Mengen wasserlöslich sind.

Als geeignete synthetische Elastomere kommen beispielsweise Polyisobutylene mit durchschnittlichen Molekulargewichten (nach GPC) von 10.000 bis 100.000 und vorzugsweise 50.000 bis 80.000, Isobutylen-Isopren-Copolymere ("Butyl Elastomere"), Styrol-Butadien-Copolymere (Styrol:Butadien-Verhältnis z.B. 1: 3 bis 3: 1), Polyvinylacetate mit durchschnittlichen Molekulargewichten (nach GPC) von 2.000 bis 90.000 und vorzugsweise 10.000 bis 65.000, Polyisoprene, Polyethylen, Vinylacetat-Vinyllaurat-Copolymere und deren Gemische. Beispiele für geeignete natürliche Elastomere sind Kautschuks wie etwa geräucherter oder flüssiger Latex oder Guayule sowie natürliche Gummistoffe wie Jelutong, Lechi caspi, Perillo, Sorva, Massaranduba balata, Massaranduba chocolate, Nispero, Rosindinba, Chicle, Gutta hang 1kang sowie deren Gemische. Die Auswahl der synthetischen und natürlichen Elastomere und deren Mischungsverhältnisse richtet sich im Wesentlichen danach, ob mit den Kaugummis Blasen erzeugt werden sollen ("bubble gums") oder nicht. Vorzugsweise werden Elastomergemische eingesetzt, die Jelutong, Chicle, Sorva und Massaranduba enthalten.

In den meisten Fällen erweisen sich die Elastomere in der Verarbeitung als zu hart oder zu wenig verformbar, so dass es sich als vorteilhaft erwiesen hat, spezielle Weichmacher mitzuverwenden, die natürlich insbesondere auch alle Anforderungen an die Zulassung als Nahrungsmittelzusatzstoffe erfüllen müssen. In dieser Hinsicht kommen vor allem Ester von Harzsäuren in Betracht, beispielsweise Ester von niederen aliphatischen Alkoholen oder Polyolen mit ganz oder teilweise gehärteten, monomeren oder oligomeren Harzsäuren. Insbesondere werden für diesen Zweck die Methyl-, Glycerin-, oder Pentareythritester sowie deren Gemische eingesetzt. Alternativ kommen auch Terpenharze in Betracht, die sich von alpha-Pinen, beta-Pinen, delta-Limonen oder deren Gemischen ableiten können.

Als Füllstoffe oder Texturiermittel kommen Magnesium- oder Calciumcarbonat, gemahlener Bimsstein, Silicate, speziell Magnesium- oder Aluminiumsilicate, Tone, Aluminiumoxide. Talkum, Titandioxid, Mono-, Di- und Tricalciumphosphat sowie Cellulosepolymere.

Geeignete Emulgatoren sind Talg, gehärteter Talg, gehärtete oder teilweise gehärtete pflanzliche Öle, Kakaobutter, Partialglyceride, Lecithin, Triacetin und gesättigte oder ungesättigte Fettsäuren mit 6 bis 22 und vorzugsweise 12 bis 18 Kohlenstoffatomen sowie deren Gemische.

Als Farbstoffe und Weißungsmittel kommen beispielsweise die für die Färbung von Lebensmitteln zugelassenen FD und C-Typen, Pflanzen- und Fruchtextrakte sowie Titandioxid in Frage.

Die Basismassen können Wachse enthalten oder wachsfrei sein; Beispiele für wachsfreie Zusammensetzungen finden sich unter anderem in der Patentschrift US 5,286,500, auf deren Inhalt hiermit ausdrücklich Bezug genommen wird.

Zusätzlich zu der wasserunlöslichen Gummibasis enthalten Kaugummizubereitungen regelmäßig einen wasserlösliche Anteil, der beispielsweise von Softener, Süßstoffen, Füllstoffen, Geschmacksstoffen, Geschmacksverstärkern, Emulgatoren, Farbstoffen, Säuerungsmitteln, Antioxidantien und dergleichen gebildet werden, hier mit der Maßgabe, dass die Bestandteile eine wenigstens hinreichende Wasserlöslichkeit besitzen. In Abhängigkeit der Wasserlöslichkeit der speziellen Vertreter können demnach einzelne Bestandteile sowohl der wasserunlöslichen wie auch der wasserlöslichen Phase angehören. Es ist jedoch auch möglich, Kombinationen beispielsweise eines wasserlöslichen und eines wasserunlöslichen Emulgators einzusetzen, wobei sich die einzelnen Vertreter, dann in unterschiedlichen Phasen befinden. Üblicherweise macht der wasserunlösliche Anteil 5 bis 95 und vorzugsweise 20 bis 80 Gew.-% der Zubereitung aus.

Wasserlösliche Softener oder Plastifiziermittel werden den Kaugummizusammensetzungen hinzugegeben um die Kaubarkeit und das Kaugefühl zu verbessern und sind in den Mischungen typischerweise in Mengen von 0,5 bis 15 Gew.-% zugegen. Typische Beispiele sind Glycerin, Lecithin sowie wässrige Lösungen von Sorbitol, gehärteten Stärkehydrolysaten oder Kornsirup.

Als Süßstoffe kommen sowohl zuckerhaltige wie zuckerfreie Verbindungen in Frage, die in Mengen von 5 bis 95, vorzugsweise 20 bis 80 und insbesondere 30 bis 60 Gew.-% bezogen auf die Kaugummizusammensetzung eingesetzt werden. Typische Saccharid-Süssstoffe sind Sucrose, Dextrose, Maltose, Dextrin, getrockneter Invertzucker, Fructose, Levulose, Galactose, Kornsirup sowie deren Gemische. Als Zuckerersatzstoffe kommen Sorbitol, Mannitol, Xylitol, gehärtete Stärkehydrolysate, Maltitol und deren Gemische in Frage. Weiterhin kommen als Zusatzstoffe auch sogenannte HIAS ("High Intensity Articifical Sweeteners") in Betracht, wie beispielsweise Sucralose, Aspartam, Acesulfamsalze, Alitam, Saccharin und Saccharinsalze, Cyclamsäure und deren Salze, Glycyrrhizine, Dihydrochalcone, Thaumatin, Monellin und dergleichen alleine oder in Abmischungen. Besonders wirksam sind auch die hydrophoben HIAS, die Gegenstand der internationalen Patentanmeldung WO 2002 091849 A1 (Wrigleys) sowie Stevia Extrakte und deren aktiven Bestandteile, insbesondere Ribeaudiosid A sind. Die Einsatzmenge dieser Stoffe hängt in erster Linie von ihrem Leistungsvermögen ab und liegt typischerweise im Bereich von 0,02 bis 8 Gew.-%.

Insbesondere für die Herstellung kalorienarmer Kaugummis eignen sich Füllstoffe wie beispielsweise Polydextrose, Raftilose, Rafitilin, Fructooligosaccharide (NutraFlora), Palatinoseoligosaaccharide, Guar Gum Hydrolysate (Sun Fiber) sowie Dextrine.

Die Auswahl an weiteren Geschmacksstoffen ist praktisch unbegrenzt und für das Wesen der Erfindung unkritisch. Üblicherweise liegt der Gesamtanteil aller Geschmacksstoffe bei 0,1 bis 15 und vorzugsweise 0,2 bis 5 Gew.-% bezogen auf die Kaugummizusammensetzung. Geeignete weitere Geschmacksstoffe stellen beispielsweise essentielle Öle, synthetische Aromen und dergleichen dar, wie etwa Anisöl, Sternanisöl, Kümmelöl, Eukalyptusöl, Fenchelöl, Citronenöl, Wintergrünöl, Nelkenöl, und dergleichen, wie sie auch beispielsweise in Mund- und Zahnpflegemittel Verwendung finden.

Die Kaugummis können des weiteren Hilfs- und Zusatzstoffe enthalten, die beispielsweise für die Zahnpflege, speziell zur Bekämpfung von Plaque und Gingivitis geeignet sind, wie z.B. Chlorhexidin, CPC oder Trichlosan. Weiter können pH-Regulatoren (z.B. Puffer oder Harnstoff), Wirkstoffe gegen Karies (z.B. Phosphate oder Fluoride), biogene Wirkstoffe (Antikörper, Enzyme, Koffein, Pflanzenextrakte) enthalten sein, solange diese Stoffe für Nahrungsmittel zugelassen sind und nicht in unerwünschter Weise miteinander in Wechselwirkung treten.

### Pharmazeutische Zubereitungen

Ein weiterer Gegenstand der Erfindung betrifft pharmazeutische Zubereitungen zum Schutz des menschlichen oder tierischen Körpers vor oxidativer Schädigung, enthaltend entweder antioxidative Zubereitungen, enthaltend
(a) Kaffeesäure,
(b) Sinapinsäure sowie gegebenenfalls
(c) Polyphenole,
oder
Extrakte enthaltend
(a) Kaffeesäure,
(b) Sinapinsäure sowie gegebenenfalls
(c) Polyphenole,
jeweils mit der Maßgabe, dass die Komponenten (a) und (b) im Gewichtsverhältnis von etwa 85:15 bis 98:2, vorzugsweise etwa 87:13 bis 97:3 zugegen sind. Soweit pharmazeutische Zubereitungen gemeint sind, gelten für diese die gleichen Überlegungen, wie sie schon für die Nahrungsergänzungsmittel oben ausgeführt worden sind. Tatsache ist, dass diese Zubereitungen eigentlich als so genannte "Neutraceuticals" oder "Cosmeceuticals" anzusehen sind und sich damit im Überschneidungsbereich von Pharmazie, Nahrungsmittel und Kosmetik befinden. Werden die Zubereitungen oral aufgenommen, spricht man in diesem Zusammenhang auch von einem "Beauty from inside" Effekt, weil dadurch einer Hautalterung vorgebeugt werden soll.

### Kosmetische Mittel

Ein weiterer Gegenstand der Erfindung betrifft kosmetische Mittel, enthaltend entweder anti-oxidative Zubereitungen, enthaltend
(a) Kaffeesäure,
(b) Sinapinsäure sowie gegebenenfalls
(c) Polyphenole,
oder
Extrakte enthaltend
(a) Kaffeesäure,
(b) Sinapinsäure sowie gegebenenfalls
(c) Polyphenole,
jeweils mit der Maßgabe, dass die Komponenten (a) und (b) im Gewichtsverhältnis von etwa 85:15 bis 98:2, vorzugsweise etwa 87:13 bis 97:3 zugegen sind.

Die erfindungsgemäßen kosmetischen Mittel können weitere typische Hilfs- und Zusatzstoffe enthalten, wie beispielsweise milde Tenside, Ölkörper, Emulgatoren, Perlglanzwachse, Konsistenzgeber, Verdickungsmittel, Überfettungsmittel, Stabilisatoren, Polymere, Siliconverbindungen, Fette, Wachse, Lecithine, Phospholipide, UV-Lichtschutzfaktoren, Feuchthaltemittel, biogene Wirkstoffe, Antioxidantien, Deodorantien, Antitranspirantien, Antischuppenmittel, Filmbildner, Quellmittel, Insektenrepellentien, Selbstbräuner, Tyrosininhibitoren (Depigmentierungsmittel), Hydrotrope, Solubilisatoren, Konservierungsmittel, Parfümöle, Farbstoffe und dergleichen enthalten. Da viele pharmazeutische Zubereitungen ähnliche Inhaltsstoffe aufweisen, gelten die nachfolgenden Beispiele hier mit.

### 1. Tenside

Als oberflächenaktive Stoffe können anionische, nichtionische, kationische und/oder amphotere bzw. zwitterionische Tenside enthalten sein, deren Anteil an den Mitteln üblicherweise bei etwa 1 bis 70, vorzugsweise 5 bis 50 und insbesondere 10 bis 30 Gew.-% beträgt. Typische Beispiele für anionische Tenside sind Seifen, Alkylbenzolsulfonate, Alkansulfonate, Olefinsulfonate, Alkylethersulfonate, Glycerinethersulfonate, α-Methylestersulfonate, Sulfofettsäuren, Alkylsulfate, Alkylethersulfate, Glycerinethersulfate, Fettsäureethersulfate, Hydroxymischethersulfate, Monoglycerid(ether)sulfate, Fettsäureamid(ether)sulfate, Mono- und Dialkylsulfosuccinate, Mono- und Dialkylsulfosuccinamate, Sulfotriglyceride, Amidseifen, Ethercarbonsäuren und deren Salze, Fettsäureisethionate, Fettsäuresarcosinate, Fettsäuretauride, N-Acylaminosäuren, wie beispielsweise Acyllactylate, Acyltartrate, Acylglutamate und Acylaspartate, Alkyloligoglucosidsulfate, Proteinfettsäurekondensate (insbesondere pflanzliche Produkte auf Weizenbasis) und Alkyl(ether)phosphate. Sofern die anionischen Tenside Polyglycoletherketten enthalten, können diese eine konventionelle, vorzugsweise jedoch eine eingeengte Homologenverteilung aufweisen. Typische Beispiele für nichtionische Tenside sind Fettalkoholpolyglycolether, Alkylphenolpolyglycolether, Fettsäurepolyglycolester, Fettsäureamidpolyglycolether, Fettaminpolyglycolether, alkoxylierte Triglyceride, Mischether bzw. Mischformale, gegebenenfalls partiell oxidierte Alk(en)yloligoglykoside bzw. Glucoronsäurederivate, Fettsäure-N-alkylglucamide, Proteinhydrolysate (insbesondere pflanzliche Produkte auf Weizenbasis), Polyolfettsäureester, Zuckerester, Sorbitanester, Polysorbate und Aminoxide. Sofern die nichtionischen Tenside Polyglycoletherketten enthalten, können diese eine konventionelle, vorzugsweise jedoch eine eingeengte Homologenverteilung aufweisen. Typische Beispiele für kationische Tenside sind quartäre Ammoniumverbindungen, wie beispielsweise das Dimethyldistearylammoniumchlorid, und Esterquats, insbesondere quaternierte Fettsäuretrialkanolaminestersalze. Typische Beispiele für amphotere bzw. zwitterionische Tenside sind Alkylbetaine, Alkylamidobetaine, Aminopropionate, Aminoglycinate, Imidazoliniumbetaine und Sulfobetaine. Bei den genannten Tensiden handelt es sich ausschließlich um bekannte Verbindungen. Typische Beispiele für besonders geeignete milde, d.h. besonders hautverträgliche Tenside sind Fettalkoholpolyglycolethersulfate, Monoglyceridsulfate, Mono- und/oder Dialkylsulfosuccinate, Fettsäureisethionate, Fettsäuresarcosinate, Fettsäuretauride, Fettsäureglutamate, α-Olefinsulfonate, Ethercarbonsäuren, Alkyloligoglucoside, Fettsäureglucamide, Alkylamidobetaine, Amphoacetale und/oder Proteinfettsäurekondensate, letztere vorzugsweise auf Basis von Weizenproteinen.

### 2. Ölkörper

Als Ölkörper kommen beispielsweise Guerbetalkohole auf Basis von Fettalkoholen mit 6 bis 18, vorzugsweise 8 bis 10 Kohlenstoffatomen, Ester von linearen C₆-C₂₂-Fettsäuren mit linearen oder verzweigten C₆-C₂₂-Fettalkoholen bzw. Ester von verzweigten C₆-C₁₃-Carbonsäuren mit linearen oder verzweigten C₆-C₂₂-Fettalkoholen, wie z.B. Myristylmyristat, Myristylpalmitat, Myristylstearat, Myristylisostearat, Myristyloleat, Myristylbehenat, Myristylerucat, Cetylmyristat, Cetylpalmitat, Cetylstearat, Cetylisostearat, Cetyloleat, Cetylbehenat, Cetylerucat, Stearylmyristat, Stearylpalmitat, Stearylstearat, Stearylisostearat, Stearyloleat, Stearylbehenat, Stearylerucat, Isostearylmyristat, Isostearylpalmitat, Isostearylstearat, Isostearylisostearat, Isostearyloleat, Isostearylbehenat, Isostearyloleat, Oleylmyristat, Oleylpalmitat, Oleylstearat, Oleylisostearat, Oleyloleat, Oleylbehenat, Oleylerucat, Behenylmyristat, Behenylpalmitat, Behenylstearat, Behenylisostearat, Behenyloleat, Behenylbehenat, Behenylerucat, Erucylmyristat, Erucylpalmitat, Erucylstearat, Erucylisostearat, Erucyloleat, Erucylbehenat und Erucylerucat. Daneben eignen sich Ester von linearen C₆-C₂₂-Fettsäuren mit verzweigten Alkoholen, insbesondere 2-Ethylhexanol, Ester von C₁₈-C₃₈-Alkylhydroxycarbonsäuren mit linearen oder verzweigten C₆-C₂₂-Fettalkoholen, insbesondere Dioctyl Malate, Ester von linearen und/oder verzweigten Fettsäuren mit mehrwertigen Alkoholen (wie z.B. Propylenglycol, Dimerdiol oder Trimertriol) und/oder Guerbetalkoholen, Triglyceride auf Basis C₆-C₁₀-Fettsäuren, flüssige Mono-/Di-/Triglyceridmischungen auf Basis von C₆-C₁₈-Fettsäuren, Ester von C₆-C₂₂-Fettalkoholen und/oder Guerbetalkoholen mit aromatischen Carbonsäuren, insbesondere Benzoesäure, Ester von C₂-C₁₂-Dicarbonsäuren mit linearen oder verzweigten Alkoholen mit 1 bis 22 Kohlenstoffatomen oder Polyolen mit 2 bis 10 Kohlenstoffatomen und 2 bis 6 Hydroxylgruppen, pflanzliche Öle, verzweigte primäre Alkohole, substituierte Cyclohexane, lineare und verzweigte C₆-C₂₂-Fettalkoholcarbonate, wie z.B. Dicaprylyl Carbonate (Cetiol^{®} CC), Guerbetcarbonate auf Basis von Fettalkoholen mit 6 bis 18, vorzugsweise 8 bis 10 C Atomen, Ester der Benzoesäure mit linearen und/oder verzweigten C₆-C₂₂-Alkoholen (z.B. Finsolv® TN), lineare oder verzweigte, symmetrische oder unsymmetrische Dialkylether mit 6 bis 22 Kohlenstoffatomen pro Alkylgruppe, wie z.B. Dicaprylyl Ether (Cetiol^{®} OE), Ringöffnungsprodukte von epoxidierten Fettsäureestern mit Polyolen, Siliconöle (Cyclomethicone, Siliciummethicontypen u.a.) und/oder aliphatische bzw. naphthenische Kohlenwasserstoffe, wie z.B. wie Squalan, Squalen oder Dialkylcyclohexane in Betracht.

### 3. Emulgatoren

Als Emulgatoren kommen beispielsweise nichtionogene Tenside aus mindestens einer der folgenden Gruppen in Frage:
- Anlagerungsprodukte von 2 bis 30 Mol Ethylenoxid und/ oder 0 bis 5 Mol Propylenoxid an lineare Fettalkohole mit 8 bis 22 C-Atomen, an Fettsäuren mit 12 bis 22 C-Atomen, an Alkylphenole mit 8 bis 15 C-Atomen in der Alkylgruppe sowie Alkylamine mit 8 bis 22 Kohlenstoffatomen im Alkylrest;
- Alkyl- und/oder Alkenyloligoglykoside mit 8 bis 22 Kohlenstoffatomen im Alk(en)ylrest und deren ethoxylierte Analoga;
- Anlagerungsprodukte von 1 bis 15 Mol Ethylenoxid an Ricinusöl und/oder gehärtetes Ricinusöl;
- Anlagerungsprodukte von 15 bis 60 Mol Ethylenoxid an Ricinusöl und/oder gehärtetes Ricinusöl;
- Partialester von Glycerin und/oder Sorbitan mit ungesättigten, linearen oder gesättigten, verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
- Partialester von Polyglycerin (durchschnittlicher Eigenkondensationsgrad 2 bis 8), Polyethylenglycol (Molekulargewicht 400 bis 5000), Trimethylolpropan, Pentaerythrit, Zuckeralkoholen (z.B. Sorbit), Alkylglucosiden (z.B. Methylglucosid, Butylglucosid, Laurylglucosid) sowie Polyglucosiden (z.B. Cellulose) mit gesättigten und/oder ungesättigten, linearen oder verzweigten Fettsäuren mit 12 bis 22 Kohlenstoffatomen und/oder Hydroxycarbonsäuren mit 3 bis 18 Kohlenstoffatomen sowie deren Addukte mit 1 bis 30 Mol Ethylenoxid;
- Mischester aus Pentaerythrit, Fettsäuren, Citronensäure und Fettalkohol und/oder Mischester von Fettsäuren mit 6 bis 22 Kohlenstoffatomen, Methylglucose und Polyolen, vorzugsweise Glycerin oder Polyglycerin.
- Mono-, Di- und Trialkylphosphate sowie Mono-, Di- und/oder Tri-PEG-alkylphosphate und deren Salze;
- Wollwachsalkohole;
- Polysiloxan-Polyalkyl-Polyether-Copolymere bzw. entsprechende Derivate;
- Block-Copolymere z.B. Polyethylenglycol-30 Dipolyhydroxystearate;
- Polymeremulgatoren, z.B. Pemulen-Typen (TR-1,TR-2) von Goodrich oder Cosmedia^{®} SP von Cognis;
- Polyalkylenglycole sowie
- Glycerincarbonat.

Im Folgenden werden besonders geeignete Emulgatoren näher erläutert:
(i) **Alkoxylate.** Die Anlagerungsprodukte von Ethylenoxid und/oder von Propylenoxid an Fettalkohole, Fettsäuren, Alkylphenole oder an Ricinusöl stellen bekannte, im Handel erhältliche Produkte dar. Es handelt sich dabei um Homologengemische, deren mittlerer Alkoxylierungsgrad dem Verhältnis der Stoffmengen von Ethylenoxid und/ oder Propylenoxid und Substrat, mit denen die Anlagerungsreaktion durchgeführt wird, entspricht. C_{12/18}-Fettsäuremono- und -diester von Anlagerungsprodukten von Ethylenoxid an Glycerin sind als Rückfettungsmittel für kosmetische Zubereitungen bekannt.
(ii) **Alkyl- und/oder Alkenyloligoglykosid.** Alkyl- und/oder Alkenyloligoglycoside, ihre Herstellung und ihre Verwendung sind aus dem Stand der Technik bekannt. Ihre Herstellung erfolgt insbesondere durch Umsetzung von Glucose oder Oligosacchariden mit primären Alkoholen mit 8 bis 18 Kohlenstoffatomen. Bezüglich des Glycosidrestes gilt, daß sowohl Monoglycoside, bei denen ein cyclischer Zuckerrest glycosidisch an den Fettalkohol gebunden ist, als auch oligomere Glycoside mit einem Oligomerisationsgrad bis vorzugsweise etwa 8 geeignet sind. Der Oligomerisierungsgrad ist dabei ein statistischer Mittelwert, dem eine für solche technischen Produkte übliche Homologenverteilung zugrunde liegt.
(iii) **Partialglyceride.** Typische Beispiele für geeignete Partialglyceride sind Hydroxystearinsäuremonoglycerid, Hydroxystearinsäurediglycerid, Isostearinsäuremonoglycerid, Isostearinsäurediglycerid, Ölsäuremonoglycerid, Ölsäurediglycerid, Ricinolsäuremoglycerid, Ricinolsäurediglycerid, Linolsäuremonoglycerid, Linolsäurediglycerid, Linolensäuremonoglycerid, Linolensäurediglycerid, Erucasäuremonoglycerid, Erucasäurediglycerid, Weinsäuremonoglycerid, Weinsäurediglycerid, Citronensäuremonoglycerid, Citronendiglycerid, Äpfelsäuremonoglycerid, Äpfelsäurediglycerid sowie deren technische Gemische, die untergeordnet aus dem Herstellungsprozeß noch geringe Mengen an Triglycerid enthalten können. Ebenfalls geeignet sind Anlagerungsprodukte von 1 bis 30, vorzugsweise 5 bis 10 Mol Ethylenoxid an die genannten Partialglyceride.
(iv) **Sorbitanester.** Als Sorbitanester kommen Sorbitanmonoisostearat, Sorbitansesquiisostearat, Sorbitan-diisostearat, Sorbitantriisostearat, Sorbitanmonooleat, Sorbitansesquioleat, Sorbitan-dioleat, Sorbitantrioleat, Sorbitanmonoerucat, Sorbitansesquierucat, Sorbitandierucat, Sorbitantrierucat, Sorbitanmonoricinoleat, Sorbitansesquiricinoleat, Sorbitandiricinoleat, Sorbitantriricinoleat, Sorbitanmonohydroxystearat, Sorbitansesquihydroxystearat, Sorbitandihydroxystearat, Sorbitantrihydroxystearat, Sorbitanmonotartrat, Sorbitansesqui-tartrat, Sorbitanditartrat, Sorbitantritartrat, Sorbitanmonocitrat, Sorbitansesquicitrat, Sorbitandicitrat, Sorbitantricitrat, Sorbitanmonomaleat, Sorbitansesquimaleat, Sorbitan-dimaleat, Sorbitantrimaleat sowie deren technische Gemische. Ebenfalls geeignet sind Anlagerungsprodukte von 1 bis 30, vorzugsweise 5 bis 10 Mol Ethylenoxid an die genannten Sorbitanester.
(v) **Polyglycerinester.** Typische Beispiele für geeignete Polyglycerinester sind Polyglyceryl-2 Dipolyhydroxystearate (Dehymuls® PGPH), Polyglycerin-3-Diisostearate (Lameform@ TGI), Polyglyceryl-4 Isostearate (Isolan® GI 34), Polyglyceryl-3 Oleate, Diisostearoyl Polyglyceryl-3 Diisostearate (Isolan® PDI), Polyglyceryl-3 Methylglucose Distearate (Tego Care® 450), Polyglyceryl-3 Beeswax (Cera Bellina®), Polyglyceryl-4 Caprate (Polyglycerol Caprate T2010/90), Polyglyceryl-3 Cetyl Ether (Chimexane® NL), Polyglyceryl-3 Distearate (Cremophor® GS 32) und Polyglyceryl Polyricinoleate (Admul® WOL 1403) Polyglyceryl Dimerate Isostearate sowie deren Gemische. Beispiele für weitere geeignete Polyolester sind die gegebenenfalls mit 1 bis 30 Mol Ethylenoxid umgesetzten Mono-, Di- und Triester von Trimethylolpropan oder Pentaerythrit mit Laurinsäure, Kokosfettsäure, Talgfettsäure, Palmitinsäure, Stearinsäure, Ölsäure, Behensäure und dergleichen.
(vi) **Anionische Emulgatoren.** Typische anionische Emulgatoren sind aliphatische Fettsäuren mit 12 bis 22 Kohlenstoffatomen, wie beispielsweise Palmitinsäure, Stearinsäure oder Behensäure, sowie Dicarbonsäuren mit 12 bis 22 Kohlenstoffatomen, wie beispielsweise Azelainsäure oder Sebacinsäure.
(vii) **Amphotere und kationische Emulgatoren.** Weiterhin können als Emulgatoren zwitterionische Tenside verwendet werden. Als zwitterionische Tenside werden solche oberflächenaktiven Verbindungen bezeichnet, die im Molekül mindestens eine quartäre Ammoniumgruppe und mindestens eine Carboxylat- und eine Sulfonatgruppe tragen. Besonders geeignete zwitterionische Tenside sind die sogenannten Betaine wie die N-Alkyl-N,N-dimethylammoniumglycinate, beispielsweise das Kokosalkyldimethylammoniumglycinat, N-Acylaminopropyl-N,N-dimethylammoniumglycinate, beispielsweise das Kokosacylaminopropyldimethyl-ammoniumglycinat, und 2-Alkyl-3-carboxylmethyl-3-hydroxyethylimidazoline mit jeweils 8 bis 18 C-Atomen in der Alkyl- oder Acylgruppe sowie das Kokosacylaminoethylhydroxyethylcarboxymethylglycinat. Besonders bevorzugt ist das unter der CTFA-Bezeichnung Cocamidopropyl Betaine bekannte Fettsäureamid-Derivat. Ebenfalls geeignete Emulgatoren sind ampholytische Tenside. Unter ampholytischen Tensiden werden solche oberflächenaktiven Verbindungen verstanden, die außer einer C_{8/18}-Alkyl- oder Acylgruppe im Molekül mindestens eine freie Aminogruppe und mindestens eine -COOH- oder -SO₃H-Gruppe enthalten und zur Ausbildung innerer Salze befähigt sind. Beispiele für geeignete ampholytische Tenside sind N-Alkylglycine, N-Alkylpropionsäuren, N-Alkylaminobuttersäuren, N-Alkyliminodipropionsäuren, N-Hydroxyethyl-N-alkylamidopropylglycine, N-Alkyltaurine, N-Alkylsarcosine, 2-Alkylaminopropionsäuren und Alkylaminoessigsäuren mit jeweils etwa 8 bis 18 C-Atomen in der Alkylgruppe.. Besonders bevorzugte ampholytische Tenside sind das N-Kokosalkylaminopropionat, das Kokosacylaminoethylaminopropionat und das C_{12/18}-Acylsarcosih. Schließlich kommen auch Kationtenside als Emulgatoren in Betracht, wobei solche vom Typ der Esterquats, vorzugsweise methylquaternierte Difettsäuretriethanolaminester-Salze, besonders bevorzugt sind.

### 4. Fette und Wachse

Typische Beispiele für Fette sind Glyceride, d.h. feste oder flüssige pflanzliche oder tierische Produkte, die im Wesentlichen aus gemischten Glycerinestern höherer Fettsäuren bestehen, als Wachse kommen u.a. natürliche Wachse, wie z.B. Candelillawachs, Carnaubawachs, Japanwachs, Espartograswachs, Korkwachs, Guarumawachs, Reiskeimölwachs, Zuckerrohrwachs, Ouricurywachs, Montanwachs, Bienenwachs, Schellackwachs, Walrat, Lanolin (Wollwachs), Bürzelfett, Ceresin, Ozokerit (Erdwachs), Petrolatum, Paraffinwachse, Mikrowachse; chemisch modifizierte Wachse (Hartwachse), wie z.B. Montanesterwachse, Sasolwachse, hydrierte Jojobawachse sowie synthetische Wachse, wie z.B. Polyalkylenwachse und Polyethylenglycolwachse in Frage. Neben den Fetten kommen als Zusatzstoffe auch fettähnliche Substanzen, wie Lecithine und Phospholipide in Frage. Unter der Bezeichnung Lecithine versteht der Fachmann diejenigen Glycero-Phospholipide, die sich aus Fettsäuren, Glycerin, Phosphorsäure und Cholin durch Veresterung bilden. Lecithine werden in der Fachwelt daher auch häufig als Phosphatidylcholine (PC). Als Beispiele für natürliche Lecithine seien die Kephaline genannt, die auch als Phosphatidsäuren bezeichnet werden und Derivate der 1,2-Diacyl-sn-glycerin-3-phosphorsäuren darstellen. Dem gegenüber versteht man unter Phospholipiden gewöhnlich Mono- und vorzugsweise Diester der Phosphorsäure mit Glycerin (Glycerinphosphate), die allgemein zu den Fetten gerechnet werden. Daneben kommen auch Sphingosine bzw. Sphingolipide in Frage.

### 5. Perlglanzwachse

Als Perlglanzwachse kommen beispielsweise in Frage: Alkylenglycolester, speziell Ethylenglycoldistearat; Fettsäurealkanolamide, speziell Kokosfettsäurediethanolamid; Partialglyceride, speziell Stearinsäuremonoglycerid; Ester von mehrwertigen, gegebenenfalls hydroxysubstituierte Carbonsäuren mit Fettalkoholen mit 6 bis 22 Kohlenstoffatomen, speziell langkettige Ester der Weinsäure; Fettstoffe, wie beispielsweise Fettalkohole, Fettketone, Fettaldehyde, Fettether und Fettcarbonate, die in Summe mindestens 24 Kohlenstoffatome aufweisen, speziell Lauron und Distearylether; Fettsäuren wie Stearinsäure, Hydroxystearinsäure oder Behensäure, Ringöffnungsprodukte von Olefinepoxiden mit 12 bis 22 Kohlenstoffatomen mit Fettalkoholen mit 12 bis 22 Kohlenstoffatomen und/oder Polyolen mit 2 bis 15 Kohlenstoffatomen und 2 bis 10 Hydroxylgruppen sowie deren Mischungen.

### 6. Kühlstoffe

Kühlstoffe sind Verbindungen, die auf der Haut ein Gefühlt der Kälte erzeugen. In der Regel handelt es sich dabei um Mentholverbindungen, die - neben dem Grundkörper Menthol selber - beispielsweise ausgewählt aus der Gruppe, die gebildet wird von Menthol Methyl Ether, Menthone Glyceryl Acetal (FEMA GRAS¹ 3807), Menthone Glyceryl Ketal (FEMA GRAS 3808), Menthyl Lactate (FEMA GRAS 3748), Menthol Ethylene Glycol Carbonate (FEMA GRAS 3805), Menthol Propylene Glycol Carbonate (FEMA GRAS 3806), Menthyl-N-ethyloxamat, Monomethyl Succinate (FEMA GRAS 3810), Monomenthyl Glutamate (FEMA GRAS 4006), Menthoxy-1,2-propanediol (FEMA GRAS 3784), Menthoxy-2-methyl-1,2-propandiol (FEMA GRAS 3849) sowie den Menthancarbonsäureestern und -amiden WS-3, WS-4, WS-5, WS-12, WS-14 und WS-30 sowie deren Gemischen.
¹ FEMA steht für "Flavor and Extracts Manufacturers Association" und GRAS ist definiert als "Generally Regarded As Safe". Eine FEMA GRAS Bezeichnung bedeutet, dass die so gekennzeichnete Substanz nach Standardmethode getestet und für toxikologisch unbedenklich erachtet wird.

Ein erster wichtiger Vertreter dieser Stoffe stellt das Monomenthyl Succinate (FEMA GRAS 3810) dar. Sowohl das Succinat als auch das analoge Monomenthyl Glutarate (FEMA GRAS 4006) stellen wichtige Vertreter von Monomenthylestern auf Basis von Di- und Polycarbonsäuren dar:

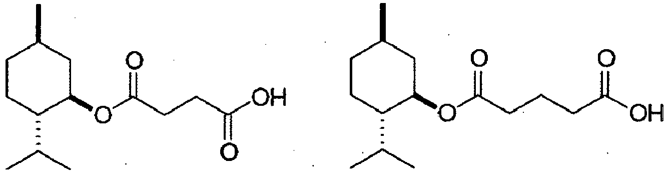

Beispiele für Anwendungen dieser Stoffe finden sich beispielsweise in den Druckschriften WO 2003 043431 (Unilever) oder EP 1332772 A1 (IFF).

Die nächste wichtige Gruppe von im Sinne der Erfindung bevorzugten Mentholverbindungen umfasst Carbonatester von Menthol und Polyolen, wie beispielsweise Glykolen, Glycerin oder Kohlenhydraten, wie beispielsweise Menthol Ethylenglycol Carbonate (FEMA GRAS 3805 = Frescolat^{®} MGC), Menthol Propylenglycol Carbonate (FEMA GRAS 3784 = Frescolat^{®} MPC), Menthol 2-Methyl-1,2-propandiol Carbonate (FEMA GRAS 3849) oder den entsprechenden Zuckerderivaten. Ebenfalls bevorzugt sind die Mentholverbindungen Menthyl Lactate (FEMA GRAS 3748 = Frescolat^{®} ML) und insbesondere das Menthone Glyceryl Acetal (FEMA GRAS 3807) bzw. Menthone Glyceryl Ketal (FEMA GRAS 3808), das unter der Bezeichnung Frescolat^{®} MGA vermarktet wird. Als ganz besonders vorteilhaft haben sich unter diesen Stoffen Menthone Glyceryl Acetal/Ketal sowie das Menthyl Lactate sowie Menthol Ethylene Glycol Carbonate bzw. Menthol Propylene Glycol Carbonatw erwiesen, die die Anmelderin unter den Bezeichnungen Frescolat^{®} MGA, Frescolat^{®} ML, Frecolat^{®} MGC und Frescolat^{®} MPC vertreibt.

In den 70er Jahren des vergangenen Jahrhunderts wurden erstmals Mentholverbindungen entwickelt, die in der 3-Stellung über eine C-C-Bindung verfügen und von denen ebenfalls eine Reihe von Vertretern eingesetzt werden können. Diese Stoffe werden im Allgemeinen als WS-Typen bezeichnet. Grundkörper ist ein Mentholderivat, bei dem die Hydroxyl- gegen eine Carboxylgruppe ersetzt ist (WS-1). Von dieser Struktur leiten sich alle weiteren WS-Typen ab, wie beispielsweise die bevorzugten Spezies WS-3, WS-4, WS-5, WS-12, WS-14 und WS-30.

### 7. Konsistenzgeber und Verdickungsmittel

Als Konsistenzgeber kommen in erster Linie Fettalkohole oder Hydroxyfettalkohole mit 12 bis 22 und vorzugsweise 16 bis 18 Kohlenstoffatomen und daneben Partialglyceride, Fettsäuren oder Hydroxyfettsäuren in Betracht. Bevorzugt ist eine Kombination dieser Stoffe mit Alkyloligoglucosiden und/oder Fettsäure-N-methylglucamiden gleicher Kettenlänge und/oder Polyglycerinpoly-12-hydroxystearaten. Geeignete Verdickungsmittel sind beispielsweise Aerosil-Typen (hydrophile Kieselsäuren), Polysaccharide, insbesondere Xanthan-Gum, Guar-Guar, Agar-Agar, Alginate und Tylosen, Carboxymethylcellulose und Hydroxyethyl- und Hydroxypropylcellulose, ferner höhermolekulare Polyethylenglycolmono- und - diester von Fettsäuren, Polyacrylate, (z.B. Carbopole® und Pemulen-Typen von Goodrich; Synthalene® von Sigma; Keltrol-Typen von Kelco; Sepigel-Typen von Seppic; Salcare-Typen von Allied Colloids), Polyacrylamide, Polymere, Polyvinylalkohol und Polyvinylpyrrolidon. Als besonders wirkungsvoll haben sich auch Bentonite, wie z.B. Bentone^{®} Gel VS-5PC (Rheox) erwiesen, bei dem es sich um eine Mischung aus Cyclopentasiloxan, Disteardimonium Hectorit und Propylencarbonat handelt. Weiter in Frage kommen Tenside, wie beispielsweise ethoxylierte Fettsäureglyceride, Ester von Fettsäuren mit Polyolen wie beispielsweise Pentaerythrit oder Trimethylolpropan, Fettalkoholethoxylate mit eingeengter Homologenverteilung oder Alkyloligoglucoside sowie Elektrolyte wie Kochsalz und Ammoniumchlorid.

### 8. Überfettungsmittel und Stabilisatoren

Als Überfettungsmittel können Substanzen wie beispielsweise Lanolin und Lecithin sowie polyethoxylierte oder acylierte Lanolin- und Lecithinderivate, Polyolfettsäureester, Monoglyceride und Fettsäurealkanolamide verwendet werden, wobei die letzteren gleichzeitig als Schaumstabilisatoren dienen.

Als Stabilisatoren können Metallsalze von Fettsäuren, wie z.B. Magnesium-, Aluminium - und/oder Zinkstearat bzw. -ricinoleat eingesetzt werden.

### 9. Polymere

Geeignete kationische Polymere sind beispielsweise kationische Cellulosederivate, wie z.B. eine quaternierte Hydroxyethylcellulose, die unter der Bezeichnung Polymer JR 400® von Amerchol erhältlich ist, kationische Stärke, Copolymere von Diallylammoniumsalzen und Acrylamiden, quaternierte Vinylpyrrolidon/Vinylimidazol-Polymere, wie z.B. Luviquat® (BASF), Kondensationsprodukte von Polyglycolen und Aminen, quaternierte Kollagenpolypeptide, wie beispielsweise Lauryldimonium Hydroxypropyl Hydrolyzed Collagen (Lamequat®L/Grünau), quaternierte Weizenpolypeptide, Polyethylenimin, kationische Siliconpolymere, wie z.B. Amodimethicone, Copolymere der Adipinsäure und Dimethylaminohydroxypropyldiethylentriamin (Cartaretine®/Sandoz), Copolymere der Acrylsäure mit Dimethyl-diallylammoniumchlorid (Merquat® 550/Chemviron), Polyaminopolyamide sowie deren vernetzte wasserlöslichen Polymere, kationische Chitinderivate wie beispielsweise quaterniertes Chitosan, gegebenenfalls mikrokristallin verteilt, Kondensationsprodukte aus Dihalogenalkylen, wie z.B. Dibrombutan mit Bisdialkylaminen, wie z.B. Bis-Dimethylamino-1,3-propan, kationischer Guar-Gum, wie z.B. Jaguar® CBS, Jaguar® C-17, Jaguar® C-16 der Firma Celanese, quaternierte Ammoniumsalz-Polymere, wie z.B. Mirapol® A-15, Mirapol® AD-1, Mirapol® AZ-1 der Firma Miranol.

Als anionische, zwitterionische, amphotere und nichtionische Polymere kommen beispielsweise Vinylacetat/Crotonsäure-Copolymere, Vinylpyrrolidon/Vinylacrylat-Copolymere, Vinylacetat/Butylmaleat/ Isobornylacrylat-Copolymere, Methylvinylether/Maleinsäureanhydrid-Copolymere und deren Ester, unvernetzte und mit Polyolen vernetzte Polyacrylsäuren, Acrylamidopropyltrimethylammoniumchlorid/ Acrylat-Copolymere, Octylacrylamid/Methylmeth-acrylat/tert.Butylaminoethylmethacrylat/2-Hydroxypropylmethacrylat-Copolymere, Polyvinylpyrrolidon,Vinylpyrrolidon/Vinylacetat-Copolymere, Vinylpyrrolidon/ Dimethylaminoethylmethacrylat/Vinylcaprolactam-Terpolymere sowie gegebenenfalls derivatisierte Celluloseether und Silicone in Frage.

### 10. Silikonverbindungen

Geeignete Siliconverbindungen sind beispielsweise Dimethylpolysiloxane, Methylphenylpolysiloxane, cyclische Silicone sowie amino-, fettsäure-, alkohol-, polyether-, epoxy-, fluor-, glykosid- und/oder alkylmodifizierte Siliconverbindungen, die bei Raumtemperatur sowohl flüssig als auch harzförmig vorliegen können. Weiterhin geeignet sind Simethicone, bei denen es sich um Mischungen aus Dimethiconen mit einer durchschnittlichen Kettenlänge von 200 bis 300 Dimethylsiloxan-Einheiten und hydrierten Silicaten handelt.

### 11. UV-Lichtschutzfaktoren

Unter UV-Lichtschutzfaktoren sind beispielsweise bei Raumtemperatur flüssig oder kristallin vorliegende organische Substanzen (Lichtschutzfilter) zu verstehen, die in der Lage sind, ultraviolette Strahlen zu absorbieren und die aufgenommene Energie in Form längerwelliger Strahlung, z.B. Wärme wieder abzugeben. Üblicherweise sind die UV-Lichtschutzfaktoren in Mengen von 0,1 bis 5 und vorzugsweise 0,2 bis 1 Gew.-% zugegen. UVB-Filter können öllöslich oder wasserlöslich sein. Als öllösliche Substanzen sind z.B. zu nennen:
- 3-Benzylidencampher bzw. 3-Benzylidennorcampher und dessen Derivate, z.B. 3-(4-Methylbenzyliden)campher beschrieben;
- 4-Aminobenzoesäurederivate, vorzugsweise 4-(Dimethylamino)benzoesäure-2-ethyl-hexylester, 4-(Dimethylamino)benzoesäure-2-octylester und 4-(Dimethylamino)benzoesäureamylester;
- Ester der Zimtsäure, vorzugsweise 4-Methoxyzimtsäure-2-ethylhexylester, 4-Methoxyzimtsäurepropylester; 4-Methoxyzimtsäureisoamylester 2-Cyano-3,3-phenylzimtsäure-2-ethylhexylester (Octocrylene);
- Ester der Salicylsäure, vorzugsweise Salicylsäure-2-ethylhexylester, Salicylsäure-4-isopropylbenzylester, Salicylsäurehomomenthylester;
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzalmalonsäure, vorzugsweise 4-Methoxybenzmalonsäuredi-2-ethylhexylester;
- Triazinderivate, wie z.B. 2,4,6-Trianilino-(p-carbo-2'-ethyl-1'-hexyloxy)-1,3,5-triazin und Octyl Triazon oder Dioctyl Butamido Triazone (Uvasorb® HEB);
- Propan-1,3-dione, wie z.B. 1-(4-tert.Butylphenyl)-3-(4'methoxyphenyl)propan-1,3-dion;
- Ketotricyclo(5.2.1.0)decan-Derivate.

Als wasserlösliche Substanzen kommen in Frage:
- 2-Phenylbenzimidazol-5-sulfonsäure und deren Alkali-, Erdalkali-, Ammonium-, Alkylammonium-, Alkanolammonium- und Glucammoniumsalze;
- 1H-Benzimidazole-4,6-Disulfonic Acid, 2,2'-(1,4-Phenylene)Bis-, Disodium Salt (Neo Heliopan^{®} AP)
- Sulfonsäurederivate von Benzophenonen, vorzugsweise 2-Hydroxy-4-methoxybenzophenon-5-sulfonsäure und ihre Salze;
- Sulfonsäurederivate des 3-Benzylidencamphers, wie z.B. 4-(2-Oxo-3-bornylidenmethyl)benzolsulfonsäure und 2-Methyl-5-(2-oxo-3-bornyliden)sulfonsäure und deren Salze.

Als typische UV-A-Filter kommen insbesondere Derivate des Benzoylmethans in Frage, wie beispielsweise 1-(4'-tert.Butylphenyl)-3-(4'-methoxyphenyl)propan-1,3-dion, 4-tert.-Butyl-4'-methoxydibenzoylmethan (Parsol® 1789), 2-(4-Diethylamino-2-hydroxybenzoyl)-benzoic acid hexylester (Uvinul® A Plus), 1-Phenyl-3-(4'-isopropylphenyl)-propan-1,3-dion sowie Enaminverbindungen. Die UV-A und UV-B-Filter können selbstverständlich auch in Mischungen eingesetzt werden. Besonders günstige Kombinationen bestehen aus den Derivate des Benzoylmethans,, z.B. 4-tert.-Butyl-4'-methoxydibenzoylmethan (Parsol® 1789) und 2-Cyano-3,3-phenylzimtsäure-2-ethyl-hexylester (Octocrylene) in Kombination mit Ester der Zimtsäure, vorzugsweise 4-Methoxyzimtsäure-2-ethylhexylester und/oder 4-Methoxyzimtsäurepropylester und/oder 4-Methoxyzimtsäureisoamylester. Vorteilhaft werden derartige Kombinationen mit wasserlöslichen Filtern wie z.B. 2-Phenylbenzimidazol-5-sulfonsäure und deren Alkali-, Erdalkali-, Ammonium-, Alkylammonium-, Alkanolammonium- und Glucammoniumsalze kombiniert.

Neben den genannten löslichen Stoffen kommen für diesen Zweck auch unlösliche Lichtschutzpigmente, nämlich feindisperse Metalloxide bzw. Salze in Frage. Beispiele für geeignete Metalloxide sind insbesondere Zinkoxid und Titandioxid und daneben Oxide des Eisens, Zirkoniums, Siliciums, Mangans, Aluminiums und Cers sowie deren Gemische. Als Salze können Silicate (Talk), Bariumsulfat oder Zinkstearat eingesetzt werden. Die Oxide und Salze werden in Form der Pigmente für hautpflegende und hautschützende Emulsionen und dekorative Kosmetik verwendet. Die Partikel sollten dabei einen mittleren Durchmesser von weniger als 100 nm, vorzugsweise zwischen 5 und 50 nm und insbesondere zwischen 15 und 30 nm aufweisen. Sie können eine sphärische Form aufweisen, es können jedoch auch solche Partikel zum Einsatz kommen, die eine ellipsoide oder in sonstiger Weise von der sphärischen Gestalt abweichende Form besitzen. Die Pigmente können auch oberflächenbehandelt, d.h. hydrophilisiert oder hydrophobiert vorliegen. Typische Beispiele sind gecoatete Titandioxide, wie z.B. Titandioxid T 805 (Degussa) oder Eusolex^{®} T2000, Eusolex^{®} T, Eusolex^{®} T-ECO, Eusolex^{®} T-S, Eusolex^{®} T-Aqua, Eusolex^{®} T-45D (alle Merck), Uvinul TiO₂ (BASF). Als hydrophobe Coatingmittel kommen dabei vor allem Silicone und dabei speziell Trialkoxyoctylsilane oder Simethicone in Frage. In Sonnenschutzmitteln werden bevorzugt sogenannte Mikro- oder Nanopigmente eingesetzt. Vorzugsweise wird mikronisiertes Zinkoxid wie z.B. Z-COTE^{®} oder Z-COTE HP1^{®} verwendet.

### 12. Feuchthaltemittel

Feuchthaltemittel dienen zur weiteren Optimierung der sensorischen Eigenschaften der Zusammensetzung sowie zur Feuchtigkeitsregulierung der Haut. Gleichzeitig wird die Kältestabilität der erfindungsgemäßen Zubereitungen, insbesondere im Falle von Emulsionen, erhöht. Die Feuchthaltemittel sind üblicherweise in einer Menge von 0,1 bis 15 Gew.-%, vorzugsweise 1 bis 10 Gew.-%, und insbesondere 5 bis 10 Gew.-% enthalten.

Erfindungsgemäß geeignet sind u.a. Aminosäuren, Pyrrolidoncarbonsäure, Milchsäure und deren Salze, Lactitol, Harnstoff und Harnstoffderivate, Harnsäure, Glucosamin, Kreatinin, Spaltprodukte des Kollagens, Chitosan oder Chitosansalze/-derivate, und insbesondere Polyole und Polyolderivate (z. B. Glycerin, Diglycerin, Triglycerin, Ethylenglycol, Propylenglycol, Butylenglycol, Erythrit, 1,2,6-Hexantriol, Polyethylenglycole wie PEG-4, PEG-6, PEG-7, PEG-8, PEG-9, PEG-10, PEG-12, PEG-14, PEG-16, PEG-18, PEG-20), Zucker und Zuckerderivate (u.a. Fructose, Glucose, Maltose, Maltitol, Mannit, Inosit, Sorbit, Sorbitylsilandiol, Sucrose, Trehalose, Xylose, Xylit, Glucuronsäure und deren Salze), ethoxyliertes Sorbit (Sorbeth-6, Sorbeth-20, Sorbeth-30, Sorbeth-40), Honig und gehärteter Honig, gehärtete Stärkehydrolysate sowie Mischungen aus gehärtetem Weizenprotein und PEG-20-Acetatcopolymer. Erfindungsgemäß bevorzugt geeignet als Feuchthaltemittel sind Glycerin, Diglycerin, Triglycerin und Butylenglycol.

### 13. Biogene Wirkstoffe und Antioxidantien

Unter biogenen Wirkstoffen sind beispielsweise Tocopherol, Tocopherolacetat, Tocopherolpalmitat, Ascorbinsäure, (Desoxy)Ribonucleinsäure und deren Fragmentierungsprodukte, β-Glucane, Retinol, Bisabolol, Allantoin, Phytantriol, Panthenol, AHA-Säuren, Aminosäuren, Ceramide, Pseudoceramide, essentielle Öle, Pflanzenextrakte, wie z.B. Prunusextrakt, Bambaranussextrakt und Vitaminkomplexe zu verstehen.

Antioxidantien unterbrechen die photochemische Reaktionskette, welche ausgelöst wird, wenn UV-Strahlung in die Haut eindringt. Typische Beispiele hierfür sind Aminosäuren (z.B. Glycin, Histidin, Tyrosin, Tryptophan) und deren Derivate, Imidazole (z.B. Urocaninsäure) und deren Derivate, Peptide wie D,L-Carnosin, D-Carnosin, L-Carnosin und deren Derivate (z.B. Anserin), Carotinoide, Carotine (z.B. α-Carotin, β-carotin, Lycopin) und deren Derivate, Chlorogensäure und deren Derivate, Liponsäure und deren Derivate (z.B. Dihydroliponsäure), Aurothioglucose, Propylthiouracil und andere Thiole (z.B. Thioredoxin, Glutathion, Cystein, Cystin, Cystamin und deren Glycosyl-, N-Acetyl-, Methyl-, Ethyl-, Propyl-, Amyl-, Butyl- und Lauryl-, Palmitoyl-, Oleyl-, γ-Linoleyl-, Cholesteryl- und Glycerylester) sowie deren Salze, Dilaurylthiodipropionat, Distearylthiodipropionat, Thiodipropionsäure und deren Derivate (Ester, Ether, Peptide, Lipide, Nukleotide, Nukleoside und Salze) sowie Sulfoximinverbindungen (z.B. Buthioninsulfoximine, Homocysteinsulfoximin, Butioninsulfone, Penta-, Hexa-, Heptathioninsulfoximin) in sehr geringen verträglichen Dosierungen (z.B. pmol bis µmol/kg), ferner (Metall)-Chelatoren (z.B. α-Hydroxyfettsäuren, Palmitinsäure, Phytinsäure, Lactoferrin), α-Hydroxysäuren (z.B. Citronensäure, Milchsäure, Äpfelsäure), Huminsäure, Gallensäure, Gallenextrakte, Bilirubin, Biliverdin, EDTA, EGTA und deren Derivate, ungesättigte Fettsäuren und deren Derivate (z.B. γ-Linolensäure, Linolsäure, Ölsäure), Folsäure und deren Derivate, Ubichinon und Ubichinol und deren Derivate, Vitamin C und Derivate (z.B. Ascorbylpalmitat, Mg-Ascorbylphosphat, Ascorbylacetat), Tocopherole und Derivate (z.B. VitaminE-acetat), Vitamin A und Derivate (Vitamin-A-palmitat) sowie Koniferylbenzoat des Benzoeharzes, Rutinsäure und deren Derivate, α-Glycosylrutin, Ferulasäure, Furfurylidenglucitol, Carnosin, Butylhydroxytoluol, Butylhydroxyanisol, Nordihydroguajakharzsäure, Nordihydroguajaretsäure, Trihydroxybutyrophenon, Harnsäure und deren Derivate, Mannose und deren Derivate, Superoxid-Dismutase, Zink und dessen Derivate (z.B. ZnO, ZnSO₄) Selen und dessen Derivate (z.B. Selen-Methionin), Stilbene und deren Derivate (z.B. Stilbenoxid, trans-Stilbenoxid) und die erfindungsgemäß geeigneten Derivate (Salze, Ester, Ether, Zucker, Nukleotide, Nukleoside, Peptide und Lipide) dieser genannten Wirkstoffe.

### 14. Hydrotrope

Zur Verbesserung des Fließverhaltens können ferner Hydrotrope, wie beispielsweise Ethanol, Isopropylalkohol, oder Polyole eingesetzt werden; diese Stoffe entsprechen weitgehend den eingangs geschildern Trägern. Polyole, die hier in Betracht kommen, besitzen vorzugsweise 2 bis 15 Kohlenstoffatome und mindestens zwei Hydroxylgruppen. Die Polyole können noch weitere funktionelle Gruppen, insbesondere Aminogruppen, enthalten bzw. mit Stickstoff modifiziert sein. Typische Beispiele sind
- Glycerin;
- Alkylenglycole, wie beispielsweise Ethylenglycol, Diethylenglycol, Propylenglycol, Butylenglycol, Hexylenglycol sowie Polyethylenglycole mit einem durchschnittlichen Molekulargewicht von 100 bis 1.000 Dalton;
- technische Oligoglyceringemische mit einem Eigenkondensationsgrad von 1,5 bis 10 wie etwa technische Diglyceringemische mit einem Diglyceringehalt von 40 bis 50 Gew.-%;
- Methyolverbindungen, wie insbesondere Trimethylolethan, Trimethylolpropan, Trimethylolbutan, Pentaerythrit und Dipentaerythrit;
- Niedrigalkylglucoside, insbesondere solche mit 1 bis 8 Kohlenstoffen im Alkylrest, wie beispielsweise Methyl- und Butylglucosid;
- Zuckeralkohole mit 5 bis 12 Kohlenstoffatomen, wie beispielsweise Sorbit oder Mannit,
- Zucker mit 5 bis 12 Kohlenstoffatomen, wie beispielsweise Glucose oder Saccharose;
- Aminozucker, wie beispielsweise Glucamin;
- Dialkoholamine, wie Diethanolamin oder 2-Amino-1,3-propandiol.

### 15. Konservierungsmittel

Als Konservierungsmittel eignen sich beispielsweise Phenoxyethanol, Formaldehydlösung, Parabene, Pentandiol oder Sorbinsäure sowie die unter der Bezeichnung Surfacine^{®} bekannten Silberkomplexe und die in Anlage 6, Teil A und B der Kosmetikverordnung aufgeführten weiteren Stoffklassen.

### 16. Parfümöle und Aromen

Als Parfümöle seien genannt Gemische aus natürlichen und synthetischen Riechstoffen. Natürliche Riechstoffe sind Extrakte von Blüten (Lilie, Lavendel, Rosen, Jasmin, Neroli, Ylang-Ylang), Stengeln und Blättern (Geranium, Patchouli, Petitgrain), Früchten (Anis, Koriander, Kümmel, Wacholder), Fruchtschalen (Bergamotte, Zitrone, Orangen), Wurzeln (Macis, Angelica, Sellerie, Kardamon, Costus, Iris, Calmus), Hölzern (Pinien-, Sandel-, Guajak-, Zedern-, Rosenholz), Kräutern und Gräsern (Estragon, Lemongras, Salbei, Thymian), Nadeln und Zweigen (Fichte, Tanne, Kiefer, Latschen), Harzen und Balsamen (Galbanum, Elemi, Benzoe, Myrrhe, Olibanum, Opoponax). Weiterhin kommen tierische Rohstoffe in Frage, wie beispielsweise Zibet und Castoreum. Typische synthetische Riechstoffverbindungen sind Produkte vom Typ der Ester, Ether, Aldehyde, Ketone, Alkohole und Kohlenwasserstoffe. Riechstoffverbindungen vom Typ der Ester sind z.B. Benzylacetat, Phenoxyethylisobutyrat, p-tert.-Butylcyclohexylacetat, Linalylacetat, Dimethylbenzylcarbinylacetat, Phenylethylacetat, Linalylbenzoat, Benzylformiat, Ethylmethylphenylglycinat, Allylcyclohexylpropionat, Styrallylpropionat und Benzylsalicylat. Zu den Ethern zählen beispielsweise Benzylethylether, zu den Aldehyden z.B. die linearen Alkanale mit 8 bis 18 Kohlenstoffatomen, Citral, Citronellal, Citronellyloxyacetaldehyd, Cyclamenaldehyd, Hydroxycitronellal, Lilial und Bourgeonal, zu den Ketonen z.B. die Jonone, α-Isomethylionon und Methylcedrylketon, zu den Alkoholen A-nethol, Citronellol, Eugenol, Isoeugenol, Geraniol, Linalool, Phenylethylalkohol und Terpineol, zu den Kohlenwasserstoffen gehören hauptsächlich die Terpene und Balsame. Bevorzugt werden jedoch Mischungen verschiedener Riechstoffe verwendet, die gemeinsam eine ansprechende Duftnote erzeugen. Auch ätherische Öle geringerer Flüchtigkeit, die meist als Aromakomponenten verwendet werden, eignen sich als Parfümöle, z.B. Salbeiöl, Kamillenöl, Nelkenöl, Melissenöl, Minzenöl, Zimtblätteröl, Lindenblütenöl, Wacholderbeerenöl, Vetiveröl, Olibanöl, Galbanumöl, Labolanumöl und Lavandinöl. Vorzugsweise werden Bergamotteöl, Dihydromyrcenol, Lilial, Lyral, Citronellol, Phenylethylalkohol, α-Hexylzimtaldehyd, Geraniol, Benzylaceton, Cyclamenaldehyd, Linalool, Boisambrene Forte, Ambroxan, Indol, Hedione, Sandelice, Citronenöl, Mandarinenöl, Orangenöl, Allylamylglycolat, Cyclovertal, Lavandinöl, Muskateller Salbeiöl, β-Damascone, Geraniumöl Bourbon, Cyclohexylsalicylat, Vertofix Coeur, Iso-E-Super, Fixolide NP, Evernyl, Iraldein gamma, Phenylessigsäure, Geranylacetat, Benzylacetat, Rosenoxid, Romilllat, Irotyl und Floramat allein oder in Mischungen, eingesetzt.

Als Aromen kommen beispielsweise Pfefferminzöl, Krauseminzöl, Anisöl, Sternanisöl, Kümmelöl, Eukalyptusöl, Fenchelöl, Citronenöl, Wintergrünöl, Nelkenöl, Menthol und dergleichen in Frage.

### 17. Farbstoffe

Als Farbstoffe können die für kosmetische Zwecke geeigneten und zugelassenen Substanzen verwendet werden, wie sie beispielsweise in der Publikation **"**Kosmetische Färbemittel" der Farbstoffkommission der Deutschen Forschungsgemeinschaft, Verlag Chemie, Weinheim, 1984, S.81-106 zusammengestellt sind. Beispiele sind Kochenillerot A (C.I. 16255), Patentblau V (C.I.42051), Indigotin (C.I.73015), Chlorophyllin (C.I.75810), Chinolingelb (C.I.47005), Titandioxid (C.I.77891), Indanthrenblau RS (C.I. 69800) und Krapplack (C.I.58000). Als Lumineszenzfarbstoff kann auch Luminol enthalten sein. Diese Farbstoffe werden üblicherweise in Konzentrationen von 0,001 bis 0,1 Gew.-%, bezogen auf die gesamte Mischung, eingesetzt.

Der Gesamtanteil der Hilfs- und Zusatzstoffe kann 1 bis 50, vorzugsweise 5 bis 40 Gew.-% - bezogen auf die Mittel - betragen. Die Herstellung der Mittel kann durch übliche Kalt - oder Heißprozesse erfolgen; vorzugsweise arbeitet man nach der Phaseninversionstemperatur-Methode.

### Gewerbliche Anwendbarkeit

Weitere Gegenstände der vorliegenden Erfindung umfassen:
- Ein erstes Verfahren zur Verbesserung der oxidativen Stabilität von Nahrungsmitteln, bei dem man diesen eine wirksame Menge der anti-oxidativen Zubereitung oder einen Extrakt, der diese enthält, zusetzt;
- Ein zweites nicht-therapeutisches Verfahren zum Schutz des menschlichen oder tierischen Körpers vor oxidativer Schädigung, bei dem man diesen mit einer wirksamen Menge der anti-oxidativen Zubereitung oder einem Extrakt, der diese enthält behandelt.
- Ein drittes Verfahren zur Verbesserung der oxidativen Stabilität von Nahrungsmitteln, bei dem man diesen eine wirksame Menge der anti-oxidativen Zubereitung oder einen Extrakt, der diese enthält, zusetzt.
- Eine erste Verwendung einer Mischung enthaltend
   (a) Kaffeesäure,
   (b) Sinapinsäure sowie gegebenenfalls
   (c) Anthocyane,
   mit der Maßgabe, dass die Komponenten (a) und (b) im Gewichtsverhältnis von etwa 85:15 bis etwa 98:2 zugegen sind, als Antioxidans.
- Eine weitere Verwendung eines Extraktes, enthaltend
   (a) Kaffeesäure,
   (b) Sinapinsäure sowie gegebenenfalls
   (c) Anthocyane,
   mit der Maßgabe, dass die Komponenten (a) und (b) im Gewichtsverhältnis von etwa 85:15 bis etwa 98:2 zugegen sind, als Antioxidans.

Es versteht sich, dass die oben im Zusammenhang mit den Zubereitungen, des Extrakten und den Mittel, die sie enthalten, genannten vorteilhaften Ausführungsformen auch für die erfindungsgemäßen Verfahren und Verwendungen mitgelten. Insofern erübrigt sich eine Wiederholung.

### Beispiele

### Beispiele 1a und 1b

### Herstellung eines Sauerkirsch-Extraktes

Ein Konzentrat eines Sauerkirschsaftes wurde mit Wasser auf einen nicht-wässrigen Anteil von 20 Gew.-% verdünnt und mit 0,1 Gew.%-iger Salzsäure auf einen pH-Wert von etwa 3 eingestellt, um die Anthocyane stabil in Lösung zu halten. Die Lösung wurde auf eine Adsorbersäule gegeben, die mit einem Adsorberharz (Beispiel 1a:Lewatit^{®} VP 0C1064, Lanxess AG, Beispiel 1b: Amberlite XAD 7) gefüllt war. Anders als bei anderen Ionenaustauscherharzen zeichnet sich dieses Säulenmaterial dadurch aus, dass sowohl sehr polare als auch unpolare Verbindungen abgereichert werden. Auf diese Weise wurden überraschenderweise insbesondere die Hydroxyzimtsäuren im angegebenen Gewichtsverhältnis sowie die Anthocyane angereichert. Die Desorption erfolgte dann mit angesäuertem wässrigem Ethanol. Anschließend wurde das Eluat beispielsweise durch Lyophilisation, Bandtrocknung oder Sprühtrocknung entwässert wird. In **Tabelle 1** ist die Zusammensetzung des Extraktes angegeben.

**Tabelle 1**

| Inhaltsstoffe des Extraktes (Mengenangaben als ppm) | | |
|---|---|---|
| **Komponenten** | **1a** | **1b** |
| Anthocyane bzw. Anthocyanidine (gesamt)* | 98.410 | 65.056 |
| Ferulasäure | <1 | 10 |
| Gallussäure | <1 | 15 |
| Kaffeesäure | 80 | 650 |
| Ellagsäure | 50 | 1.200 |
| p-Cumarsäure | 70 | 120 |
| Chlorogensäure | 2800 | 13.800 |
| Sinapinsäure | 5 | 20 |
| Maltose | k.A. | 900 |
| ***Gewichisverhältnis Kaffeesäure : Sinapinsäure*** | | ***97:3*** |

| | | |
|---|---|---|
| *berechnet als Cyanidin-3-O-Glucosid | | |

### Beispiele 2 bis 4, Vergleichsbeispiele V1 bis V3

### Bestimmung der anti-oxidativen Wirkung

Die Bestimmung der anti-oxidativen Wirkung und der Synergie zwischen den einzelnen Komponenten erfolgte nach der OARC -Fluorescein Methode, bei der Inhibierungszeit und der Grad der Inhibierung in einen Messwert einfließen. Die Messungen wurden auf dem Gerät Synergy H4 der Firma BioTek Inc. durchgeführt, die berechneten Werte wurden einer Kalibrierkurve gegenüber Trolox^{®} als Standard entnommen. Das Verfahren ist beispielsweise in denr Veröffentlichungen von Prior et. al., J. Agric Food. Chem. 50, S. 4437-4444 (2000**)** sowie Davalos et. al, ibid 52, S. 58-54 (2004**)** ausführlich beschrieben. Diese Schriften werden bezüglich der Beschreibung der Messmethode voll umfänglich über Bezugnahme in die Patentanmeldung aufgenommen. Die Ergebnisse sind in **Tabelle 2** zusammengefasst.

**Tabelle 2**

| Gemessene und berechnete ORAC-Werte | | | | | | |
|---|---|---|---|---|---|---|
| **Bsp.** | **Kaffeesäure** | **Sinapinsäure** | **Gemessen** | **Berechnet** | **Δ** | **Δ[%]** |
| V1 | 100 Gew.-Teile | - | 21.587 | - | - | - |
| V2 | - | 100 Gew.-Teile | 11.459 | - | - | - |
| V3 | 50 Gew.-Teile | 50 Gew.-Teile | 16.627 | 16.523 | 104 | 0,62 |
| 2 | 97 Gew.-Teile | 3 Gew.-Teile | 22.245 | 21.284 | 961 | 4,52 |
| 3 | 93 Gew.-Teile | 7 Gew.-Teile | 21.757 | 20.863 | 894 | 4.29 |
| 4 | 87 Gew.-Teile | 13 Gew.-Teile | 21.578 | 20.321 | 1.258 | 6.19 |

Die zwischen den berechneten und gemessenen ORAC-Werten gefundene positive Differenz belegt, dass diese Mischungen eine synergistische Verstärkung in der oxidativen Wirkung zeigen. Mit 4 bis 6 % ist die Abweichung auch signifikant.

### Beispiele 5 bis 7, Vergleichsbeispiel V4

### Wirksamkeit gegen freie Radikale

Die Wirksamkeit der Testsubstanzen gegen freie Radikale wurde nach verschiedenen Methoden sowohl chemisch als auch biochemisch untersucht:
- Methode A: In einem ersten Verfahren wurde Diphenylpicrylhydrazyl (DPPH°) eingesetzt, ein verhältnismäßig stabiles Radikal, welches eine purpurfarbene Lösung ergibt. Bestimmt wurde die optische Dichte (DO) bei 513 nm.
- Methode B: In Gegenwart von Eisen(II)-ionen und EDTA wurden aus Wasserstoffperoxid Hydroxylradikale freigesetzt und zur Oxidation von Desoxyribose verwendet. Das Oxidationsprodukt bildet mit Thiobarbitursäure eine pinkfarbene Verbindung. Dessen Konzentration korrespondiert mit der optischen Dichte bei 532 nm. Untersucht wurde, ob in Gegenwart der Testprodukte weniger Desoxyribose oxidiert, d.h. weniger freie Radikale freigesetzt werden.
- Methode C: Der zuvor geschilderte Versuch wurde in Abwesenheit von EDTA untersucht um die Eignung der Testsubstanzen, inaktive Eisenkomplexe zu bilden, zu überprüfen.
- Methode D: Xanthin Oxidase ist ein Enzym welches infolge oxidativen Stresses ausgeschüttet wird und den Zerfall der Purinbasen Adenin und Guanin in Uronsäure und Superoxidanionen katabolisiert. Letztere dismutieren spontan oder in Gegenwart von Superoxid Dismutase in Wasserstoffperoxid und Sauerstoff. Die Menge an Superoxidanion kann durch NBT Reduktion über die optische Dichte bei 490 nm bestimmt werden. Untersucht wurde, ob in Gegenwart der Testsubstanzen weniger Superoxidanionen generiert bzw. mehr Anionen zerstört werden.

Eingesetzt wurden Sauerkrisch-Extrakte mit standardisiertem Anthocyangehalt und einem Gehalt von Kaffee- und Sinapinsäure von 700 ppm. Die Extrakte unterschieden sich jedoch im Gewichtsverhältnis, in dem die beiden Säuren vorhanden waren. Die Ergebnisse sind in **Tabelle 3** zusammengefasst.

**Tabelle 3**

| Wirkung gegen freie Radikale (Angabe zu A in optischer Dichte, übrige Angabe in %-rel.) | | | | | | |
|---|---|---|---|---|---|---|
| **Bsp.** | **Kaffeesäure** | **Sinapinsäure** | **Testmethode** | | | |
| | | | **A** | **B** | **C** | **D** |
| V4 | 50 Gew.-Teile | 50 Gew.-Teile | 0,0013 | 0,26 | 0,9 | 0,59 |
| 4 | 97 Gew.-Teile | 3 Gew.-Teile | 0,0019 | 0,23 | 0,9 | 0,54 |
| 5 | 93 Gew.-Teile | 7 Gew.-Teile | 0,0023 | 0,22 | 0,8 | 0,53 |
| 7 | 87 Gew.-Teile | 13 Gew.-Teile | 0,0026 | 0,21 | 0,8 | 0,52 |

Die Ergebnisse lassen den Schluss zu, dass die Testsubstanzen eine antioxidative Wirksamkeit besitzen, die deutlich über der eines Extraktes mit einer 1:1 Mischung aus Kaffee- und Sinapinsäure liegt. Die Testsubstanzen besitzen in Abwesenheit von EDTA ein besonders hohes Potential zum Quenchen von Hydroxylionen, wodurch nachgewiesen ist, dass sie stabile Eisenkomplexe bilden. Schließlich besitzen sie ein starkes Potential, die Bildung von Superoxidanionen zu verhindern.

Die Erfindung wird im Folgenden an Hand von zahlreichen Formulierungsbeispielen weiter erläutert ohne damit darauf eingeschränkt zu sein.

**Tabelle I**

| Erfrischungsgetränke (Mengenangaben als Gew.-%) | | | | | | | |
|---|---|---|---|---|---|---|---|
| **Inhaltsstoffe** | **A** | **B** | **C** | **D** | **E** | **F** | **G** |
| Sucrose | 10 | 10 | 7 | - | - | 8 | 7 |
| Glucose/Fructose Sirup | - | - | - | - | 10 | - | - |
| Rebaudiosid A 95 % | - | - | 0,02 | 0,05 | - | - | - |
| Zitronensäure | 0,15 | 0,15 | 0,06 | 0,15 | 0,15 | 0,15 | 0,15 |
| Phosphorsäure | - | - | 0,07 | - | - | - | - |
| Zuckercoleur | - | - | 0,14 | - | - | - | - |
| Koffein | - | - | 0,01 | - | - | - | - |
| Zitrusaroma | 0,1 | 0,05 | - | 0,1 | 0,1 | 0,1 | 0,1 |
| Limonenaroma | - | 0,05 | - | - | - | - | - |
| Getränkeemulsion Typ "Cola" | - | - | 0,05 | - | - | - | - |
| Phloretin | - | - | 0,002 | 0,003 | - | 0,002 | 0,001 |
| Hesperetin | - | - | 0,001 | 0,002 | - | - | 0,002 |
| Sauerkirsch-Extrakt nach Beispiel 1 | 0,1 | 0,1 | 0,1 | 0,1 | 0,1 | 0,1 | 0,1 |
| Homoeriodictyol-Na | - | - | 0,005 | 0,005 | - | - | - |
| Wasser | Ad 100 | | | | | | |

Die Zutaten wurden in der angegebenen Reihenfolge gemischt und mit Wasser auf 100 % aufgefüllt. Die Mischungen werden in Glasflaschen gefüllt und carbonisiert.

**Table II**

| Hartkaramellen (Mengenangaben als Gew.-%) | | | | |
|---|---|---|---|---|
| **Inhaltsstoffe** | **A** | **B** | **C** | **D** |
| Zucker | 74,50 | - | - | - |
| Palatinit, Type M | - | 74,00 | 75,50 | 75,00 |
| Zitronensäure | 0,5 | 1,0 | 0,5 | - |
| Farbstoff, gelb | - | 0,01 | - | - |
| Farbstoff, rot | - | - | 0,01 | - |
| Farbstoff, blau | 0,01 | - | - | 0,01 |
| Pfefferminzaroma | 0,1 | - | - | 0,1 |
| Zitrusaroma | - | 0,1 | - | - |
| Rote Beeren-Aroma | - | - | 0,1 | - |
| Rebaudiosid A 98 % | - | 0,040 | - | 0,040 |
| Balansin A | - | 0,005 | 0,010 | 0,005 |
| Hesperetin | - | 0,001 | - | 0,001 |
| Phloretin | - | 0,002 | - | - |
| Sauerkirsch-Extrakt nach Beispiel 1 | 0,02 | 0,01 | 0,02 | 0,02 |
| Wasser | ad 100 | ad 100 | ad 100 | ad 100 |

**Tabelle III**

| Joghurt mit niedrigem Fettgehalt (Mengenangaben als Gew.-%) | | | | |
|---|---|---|---|---|
| **Inhaltsstoffe** | **A** | **B** | **C** | **D** |
| Sucrose | 10 | 8 | 6 | - |
| Rebaudiosid A 98 % | - | - | - | 0,050 |
| Sauerkirsch-Extrakt nach Beispiel 1 | 0,2 | 0,1 | 0,2 | 0,2 |
| Hesperetin | - | 0,001 | 0,001 | 0,002 |
| Phloretin | - | - | 0,002 | 0,002 |
| Homoeriodictyol-Natriumsalz | - | - | - | 0,005 |
| Joghurt, 0,1 % Fett | zu 100 % auffüllen | | | |

**Tabelle IV**

| Fruchtgummis (Mengenangaben als Gew.-%) | | |
|---|---|---|
| **Inhaltsstoffe** | **A** | **B** |
| Saccharose | 34, 50 | 8,20 |
| Glucosesirup, DE 40 | 31,89 | 30,09 |
| Iso Sirup C* Tru Sweet 01750 (Cerestar GmbH) | 1,50 | 2,10 |
| Gelatine 240 Bloom | 8,20 | 9,40 |
| Polydextrose (Litesse^{®} Ultra, Danisco Cultor GmbH) | - | 24,40 |
| Farbstoff | 0,01 | 0,01 |
| Zitrusaroma | 0,20 | |
| Kirscharoma | - | 0,10 |
| Sauerkirsch-Extrakt nach Beispiel 1 | 0,2 | 0,1 |
| Wasser | ad 100 | ad 100 |

**Table V**

| Zuckerfreies Kaugummi (Mengenangaben als Gew.-%) | |
|---|---|
| **Inhaltssoffe** | **Gehalt** |
| Kaugummibasis | 30.00 |
| Sorbitolpulver | Ad 100 |
| Palatinit | 9.50 |
| Xylitol | 2.00 |
| Mannitol | 3.00 |
| Aspartam | 0.10 |
| Acesulfam K | 0.10 |
| Emulgum / Emulgator | 0.30 |
| Sorbitol 70 %. in Wasser | 14.00 |
| Glycerin | 1.00 |
| Pfefferminzaroma | 1.50 |
| Sauerkirsch-Extrakt nach Beispiel 1 | 0.20 |

**Tabelle VI**

| Sonnenschutzmittel (Wasser, Konservierungsmittel ad 100 Gew.-%) | | | | | | | | | | |
|---|---|---|---|---|---|---|---|---|---|---|
| **Zusammensetzung (INCI)** | **A** | **B** | **C** | **D** | **E** | **F** | **G** | **H** | **I** | **J** |
| **Dehymuls® PGPH** | 4,0 | 3,0 | - | 5,0 | - | - | - | - | - | - |
| Polyglyceryl-2 Dipolyhydroxystearate | | | | | | | | | | |
| **Lameform® TGI** | 2,0 | 1,0 | - | - | - | - | - | - | - | - |
| Polyglyceryl-3 Diisostearate | | | | | | | | | | |
| **Emulgade® PL 68/50** | - | - | - | - | 4,0 | - | - | - | 3,0 | - |
| Cetearyl Glucoside (and) Cetearyl Alcohol | | | | | | | | | | |
| **Eumulgin®B2** | - | - | - | - | - | - | - | 2,0 | - | - |
| Ceteareth-20 | | | | | | | | | | |
| **Tegocare® PS** | - | - | 3,0 | - | - | - | 4,0 | - | - | - |
| Polyglyceryl-3 Methylglucose Distearate | | | | | | | | | | |
| **Eumulgin VL 75** | - | - | - | - | - | 3,5 | - | - | 2,5 | - |
| Polyglyceryl-2 Dipolyhydroxystearate (and) Lauryl Glucoside (and) Glycerin | | | | | | | | | | |
| **Bees Wax** | 3,0 | 2,0 | 5,0 | 2,0 | - | - | - | - | - | - |
| **Cutina® GMS** | - | - | - | - | - | 2,0 | 4,0 | - | - | 4,0 |
| Glyceryl Stearate | | | | | | | | | | |
| **Lanette® O** | - | - | 2,0 | - | 2,0 | 4,0 | 2,0 | 4,0 | 4,0 | 1,0 |
| Cetearyl Alcohol | | | | | | | | | | |
| **Antaron® V 216** | - | - | - | - | - | 3,0 | - | - | - | 2,0 |
| PVP / Hexadecene Copolymer | | | | | | | | | | |
| **Myritol® 818** | 5,0 | - | 10,0 | - | 8,0 | 6,0 | 6,0 | - | 5,0 | 5,0 |
| Cocoglycerides | | | | | | | | | | |
| **Finsolv® TN** | - | 6,0 | - | 2,0 | - | - | 3,0 | - | - | 2,0 |
| C12/15 Alkyl Benzoate | | | | | | | | | | |
| **Cetiol® J 600** | 7,0 | 4,0 | 3,0 | 5,0 | 4,0 | 3,0 | 3,0 | - | 5,0 | 4,0 |
| Oleyl Erucate | | | | | | | | | | |
| **Cetiol® OE** | 3,0 | - | 6,0 | 8,0 | 6,0 | 5,0 | 4,0 | 3,0 | 4,0 | 6,0 |
| Dicaprylyl Ether | | | | | | | | | | |
| **Mineral Oil** | - | 4,0 | - | 4,0 | - | 2,0 | - | 1,0 | - | - |
| **Cetiol® PGL** | - | 7,0 | 3,0 | 7,0 | 4,0 | - | - | - 1,0 | 1,0 | - |
| Hexadecanol (and) Hexyldecyl Laurate | | | | | | | | | | |
| **Bisabolol** | 1,2 | 1,2 | 1,2 | 1,2 | 1,2 | 1,2 | 1,2 | 1,2 | 1,2 | 1,2 |
| **Sauerkirschextrakt nach Beispiel 1** | 0,2 | 0,2 | 0,2 | 0,2 | 0,2 | 0,2 | 0,2 | 0,2 | 0,2 | 0,2 |
| **Hydagen® CMF** | 1,0 | 1,0 | 1,0 | 1,0 | 1,0 | 1,0 | 1,0 | 1,0 | 1,0 | 1,0 |
| Chitosan | | | | | | | | | | |
| **Cophenol® F 1300** | 0,5 | 1,0 | 1,0 | 2,0 | 1,0 | 1,0 | 1,0 | 2,0 | 0,5 | 2,0 |
| Tocopherol /Tocopheyl Acetate | | | | | | | | | | |
| **Neo Hellopan® Hydro** | 3,0 | - | - | 3,0 | - | - | 2,0 | - | 2,0 | - |
| Sodium Phenylbenzimidazole Sulfonate | | | | | | | | | | |
| **Neo Heliopan® 303** | - | 5,0 | - | - | - | 4,0 | 5,0 | - | - | 10,0 |
| Octocrylene | | | | | | | | | | |
| **Neo Heliopan® BB** | 1,5 | - | - | 2,0 | 1,5 | - | - | - | 2,0 | - |
| Benzophenone-3 | | | | | | | | | | |
| **Neo Heliopan® E 1000** | 5,0 | - | 4,0 | - | 2,0 | 2,0 | 4,0 | 10,0 | - | - |
| Isoamyl p-Methoxycinnamate | | | | | | | | | | |
| **Neo Heliopan® AV** | 4,0 | - | 4,0 | 3,0 | 2,0 | 3,0 | 4,0 | - | 10,0 | 2,0 |
| Octyl Methoxycinnamate | | | | | | | | | | |
| **Uvinul® T 150** | 2,0 | 4,0 | 3,0 | 1,0 | 1,0 | 1,0 | 4,0 | 3,0 | 3,0 | 3,0 |
| Octyl Triazone | | | | | | | | | | |
| **Zinc Oxide** | - | 6,0 | 6,0 | - | 4,0 | - | - | - | - | 5,0 |
| **Titanium Dioxide** | - | - | - | - | - | - | - | 5,0 | - | - |
| **Glycerin (86 Gew.-%ig)** | 5,0 | 5,0 | 5,0 | 5,0 | 5,0 | 5,0 | 5,0 | 5,0 | 5,0 | 5.0 |

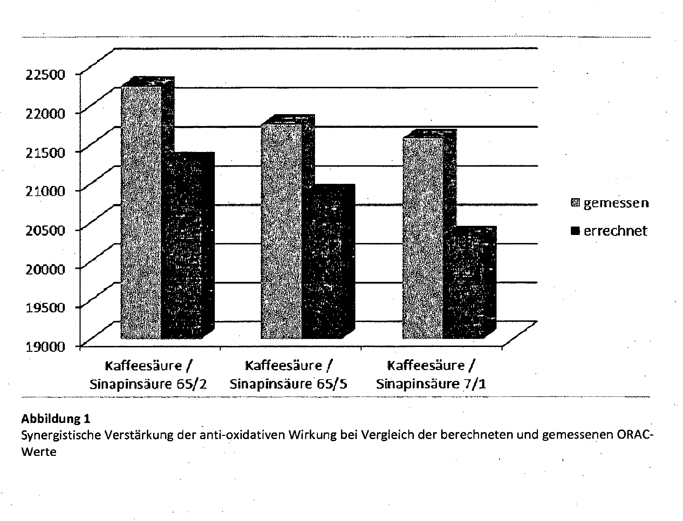

## Patentansprüche

1. Extrakt, enthaltend
(a) Kaffeesäure,
(b) Sinapinsäure sowie gegebenenfalls
(c) Polyphenole,
mit der Maßgabe, dass die Komponenten (a) und (b) im Gewichtsverhältnis von etwa 85:15 bis etwa 98:2 zugegen sind.

2. Extrakt nach Anspruch 1, **dadurch gekennzeichnet**, das er
(a) 150 bis 1.000 ppm Kaffeesäure,
(b) 10 bis 50 ppm Sinapinsäure, und
(c) 10 bis 60 Gew.-% Polyphenole sowie
ad 100 Gew.-% im Wesentlichen Lösungsmittel enthält.

3. Extrakt nach Anspruch 1, **dadurch gekennzeichnet, dass** er einen Gesamtgehalt an Hydroxyzimtsäuren von etwa 0,5 bis etwa 3 Gew.-% aufweist.

4. Extrakt nach Anspruch 1, **dadurch gekennzeichnet, dass** er aus Pflanzen oder Früchten gewonnen wird, die ausgewählt sind aus der Gruppe, die gebildet wird von Açai (*Euterpe oleracea*)*;* Aronia (e.g. *Aronia arbutifolia, Aronia melanocarpa; Aronia prunifolia*); Schwarze und Rote Johannisbeeren (e.g. *Ribes rubrum, Ribes spciatum, Ribes alpinum, Ribes schlechtendalii, Ribes multiflorum, Ribes petraeum, Ribes trite, Ribes nigrum*); Brombeeren (*Rubus sp*.); Schwarze Karotten (*Daucus carota*); Schwarze Tomaten (*Solanum lycopersicum*); Blutorangen (*Citrus sinensis*); Blaubeeren (*Vacciniumcorymbosum, Vaccinium angustifolium*); Schwarzdorn (*Prunus spinosa*); Rauschbeeren (*Vaccinium ulginosum*); Moltebeeren (*Rubus chamaemorus*); Preiselbeeren (e.g. *Vaccinium oxycoccos, Vaccinium microcarpus, Vaccinium macrocarpus, Vaccinium erythrocarpus);* Krähenbeeren (e.g. *Empetrum nigrum, Empetrum eamesii, Empetrum rumbrum, Empetrum hermaphroditum*); Holunderbeeren (e.g. *Sambucus nigra, Sambucus racemo*sa); Hibiskus (*Hibiscus sabdariffa, Roselle*); Kronsbeeren (e.g. *Vaccinium vitus idaea*); Magellanbeeren ("Calafate", *Berberis microphylla, Breberis buxifolia*); Makibeeren (e.g. *Aristocelia chilensis*); Bergbeeren (*Vaccinium membranaceum*); Pflaumen und Zwetschgen (*Prunus domestica*)*;* Himbeeren (*Rubus idaeus, Rubus occidentalis*)*;* Rote Stachelbeeren (*Ribes uva-crispa, Ribes grossularia*)*;* Rote Trauben (*Vitis vinifera, Vitis labrusca, Vitis riparia, Vitis rotundifolia*)*;* Rowanbeeren (*Sorbus aucuparia);* Sauerkirschen (*Prunus avium, Prunus cerasus*)*;* Erdbeeren (*Fragaria ananassa*)*;* Süßkirschen (*Prunus avium);* Taybeeren (*Rubus X).*

5. Sauerkirschextrakt nach Anspruch 4, **dadurch gekennzeichnet, dass** er
(a) einen ORAC-Wert von größer 5.000 und/oder
(b) einen Polyphenolgehalt von größer 40 Gew.-% und/oder
(c) einen Anthocyangehalt von etwa 6 bis etwa 25 Gew.-% und/oder
(d) einen Aschegehalt von kleiner 3 Gew.-% und/oder
(e) einen Zuckergehalt von kleiner 0,25 Gew.-%
aufweist.

6. Verfahren zur Herstellung eines Extraktes nach Anspruch 1, bei dem man
(i) aus den Pflanzen oder Früchten einen Saft oder Auszug herstellt und ansäuert,
(ii) das angesäuerte Produkt über eine Adsorbersäule gibt, die als Absorptionsmittel ein makroporöses vernetztes nicht-funktionalisiertes Polystyrolharz enthält,
(iii) die Mischung enthaltend die Komponenten (a), (b) und (c) mit wässrigem Alkohol desorbiert und schließlich
(iv) das Eluat trocknet.

7. Nahrungs- oder Nahrungsergänzungsmittel, enthaltend den Extrakt nach Anspruch 1.

8. Pharmazeutische Zubereitung zum Schutz des menschlichen oder tierischen Körpers vor oxidativer Schädigung, enthaltend den Extrakt nach Anspruch 1.

9. Kosmetisches Mittel, enthaltend den Extrakt nach Anspruch 1.

10. Verfahren zur Verbesserung der oxidativen Stabilität von Nahrungsmitteln, bei dem man diesen eine wirksame Menge eines Extrakts nach Anspruch 1 zusetzt.

11. Verwendung eines Extraktes, enthaltend
(a) Kaffeesäure,
(b) Sinapinsäure sowie gegebenenfalls
(c) Polyphenole,
mit der Maßgabe, dass die Komponenten (a) und (b) im Gewichtsverhältnis von etwa 85:15 bis etwa 98:2 zugegen sind, als Antioxidans.

## Claims

1. An extract, comprising
(a) caffeic acid,
(b) sinapinic acid and, optionally,
(c) polyphenols,
with the proviso that components (a) and (b) are present in a weight ratio of about 85:15 to about 98:2.

2. The extract according to claim 1, **characterised in that** it comprises
(a) 150 to 1.000 ppm caffeic acid,
(b) 10 to 50 ppm sinapinic acid, and
(c) 10 to 60 % by weight polyphenols, and
is essentially filled up to 100 % by weight with solvents.

3. The extract according to claim 1, **characterised in that** it has a total content of hydroxycinnamic acids of about 0.5 to about 3 % by weight.

4. The extract according to claim 1, **characterised in that** it is obtained from plants or fruits selected from the group consisting of Açai (*Euterpe oleracea*); Aronia (e.g. *Aronia arbutifolia, Aronia melanocarpa; Aronia prunifolia*); black currants and red currants (e.g. *Ribes rubrum, Ribes spciatum, Ribes alpinum, Ribes schlechtendalii, Ribes multiflorum, Ribes petraeum, Ribes trite, Ribes nigrum*); blackberries (*Rubus sp*.); black carrots (*Daucus carota*); black tomatoes (*Solanum lycopersicum*); blood oranges (*Citrus sinensis*); blueberries (*Vaccinium corymbosum, Vaccinium angustifolium*); black haw (*Prunus spinosa*); bilberries (V*accinium ulginosum*); cloudberries (*Rubus chamaemorus*); lingonberries (e.g. *Vaccinium oxycoccos, Vaccinium microcarpus, Vaccinium macrocarpus, Vaccinium erythrocarpus);* black crowberries (e.g. *Empetrum nigrum, Empetrum eamesii, Empetrum rumbrum, Empetrum hermaphroditum*); elderberries (e.g. *Sambucus nigra, Sambucus racemosa*); hibiscus (*Hibiscus sabdariffa, Roselle*); cowberries (e.g. *Vaccinium vitus idaea*); Magellan barberries ("Calafate", *Berberis microphylla, Breberis buxifolia*); Maqui berries (e.g. *Aristotelia chilensis*); mountain huckleberries (*Vaccinium membranaceum*); plums and damsons (*Prunus domestica*); raspberries (*Rubus idaeus, Rubus occidentalis*); red gooseberries (*Ribes uva-crispa, Ribes grossularia*); red grapes (*Vitis vinifera, Vitis labrusca, Vitis riparia, Vitis rotundifolia*); rowanberries (*Sorbus aucuparia*); sour cherries (*Prunus avium, Prunus cerasus*); strawberries (*Fragaria ananassa*); sweet cherries (*Prunus avium*); tay berries (*Rubus X*).

5. A sour cherry extract according to claim 4, **characterized in that** it has
(a) an ORAC value of above 5,000 and/or
(b) a polyphenol content of above 40 % by weight and/or
(c) an anthocyanine content of about 6 to about 25 % by weight and/or
(d) an ash content of less than 3 % by weight and/or
(e) a sugar content of less than 0.25 % by weight.

6. The method of production of an extract according to claim 1, wherein
(i) a juice or an extract is produced from the plants or fruits, which is then acidified,
(ii) the acidified product is passed over an adsorber column which contains as adsorption means a macroporous, crosslinked, non-functionalized polystyrene resin,
(iii) the mixture comprising components (a), (b) and (c) is desorbed by means of aqueous alcohol and finally
(iv) the eluate is dried.

7. A food or a dietary supplement, comprising the extract according to claim 1.

8. A pharmaceutical composition for the protection of the human or animal body against oxidative damage, comprising the extract according to claim 1.

9. A cosmetic means, comprising the extract according to claim 1.

10. A method to improve the oxidative stability of food, wherein an effective amount of the extract according to claim 1 is added to the food.

11. Use of a extract comprising
(a) caffeic acid,
(b) sinapinic acid and, optionally,
(c) polyphenols,
with the proviso that components (a) and (b) are present as an antioxidant in a weight ratio of about 85:15 to about 98:2.

## Revendications

1. Extrait, contenant
(a) de l'acide caféique,
(b) de l'acide sinapique ainsi qu'éventuellement
(c) des polyphénols,
étant entendu que les composants (a) et (b) sont présents en un rapport pondéral d'environ 85:15 à environ 98:2.

2. Extrait selon la revendication 1, **caractérisé en ce qu'**il contient
(a) 150 à 1000 ppm d'acide caféique,
(b) 10 à 50 ppm d'acide sinapique et
(c) 10 à 60 % en poids de polyphénols ainsi que
en complément à 100 % en poids essentiellement un solvant.

3. Extrait selon la revendication 1, **caractérisé en ce qu'**il présente une teneur totale en acides hydroxycinnamiques d'environ 0,5 à environ 3 % en poids.

4. Extrait selon la revendication 1, **caractérisé en ce qu'**il est obtenu à partir de plantes ou de fruits qui sont choisis dans le groupe qui est constitué par le palmier pinot (*Euterpe oleracea*); les aronias (par exemple *Aronia arbutifolia, Aronia melanocarpa; Aronia prunifolia*); les cassis et groseilles (par exemple *Ribes rubrum, Ribes spicatum, Ribes alpinum, Ribes schlechtendalii, Ribes multiflorum, Ribes petraeum, Ribes trite, Ribes nigrum*); les mûres (*Rubus sp*.); les carottes noires (*Daucus carota*); les tomates noires (*Solanum lycopersicum*); les oranges sanguines (*Citrus sinensis*); les myrtilles (Vacci*nium corymbosum, Vaccinium angustifolium*); le prunellier (*Prunus spinosa*); les myrtilles des marais (*Vaccinium ulginosum*); les plaquebières (*Rubus chamaemorus*); les airelles rouges (par exemple *Vaccinium oxycoccos, Vaccinium microcarpus, Vaccinium macrocarpus, Vaccinium erythrocarpus*); les camarines (par exemple *Empetrum nigrum, Empetrum eamesii, Empetrum rubrum, Empetrum hermaphroditum*); les baies de sureau (par exemple *Sambucus nigra, Sambucus racemosa*); l'hibiscus (*Hibiscus sabdariffa, Roselle*); les petites airelles (par exemple *Vaccinium vitis-idaea*); les berbéris (« calafate », *Berberis microphylla, Berberis buxifolia*); les baies de maqui (par exemple *Aristotelia chilensis*); les airelles à feuilles membraneuses (*Vaccinium membranaceum*); les prunes et les quetsches (*Prunus domestica*); les framboises (*Rubus idaeus, Rubus occidentalis*); les groseilles à maquereau (*Ribes uva-crispa, Ribes grossularia*); les raisins rouges (*Vitis vinifera, Vitis labrusca, Vitis riparia, Vitis rotundifolia*); les baies de sorbier (*Sorbus aucuparia*); les griottes (*Prunus avium, Prunus cerasus*); les fraises (*Fragaria ananassa*); les cerises douces (*Prunus avium*); les mûres-framboises (*Rubus X*).

5. Extrait de griottes selon la revendication 4, **caractérisé en ce qu'**il présente
(a) une valeur ORAC de plus de 5 000 et/ou
(b) une teneur en polyphénols de plus de 40 % en poids et/ou
(c) une teneur en anthocyanes d'environ 6 à environ 25 % en poids et/ou
(d) une teneur en cendres de moins de 3 % en poids et/ou
(e) une teneur en sucres de moins de 0,25 % en poids.

6. Procédé pour la préparation d'un extrait selon la revendication 1, dans lequel
(i) à partir de plantes ou de fruits on prépare et acidifie un jus ou extrait,
(ii) on injecte le produit acidifié sur une colonne d'adsorbant qui contient comme adsorbant une résine polystyrène macroporeuse réticulée non fonctionnalisée,
(iii) on désorbe le mélange contenant les composants (a), (b) et (c) avec un alcool aqueux et enfin
(iv) on sèche l'éluat.

7. Produit alimentaire ou complément nutritionnel, contenant l'extrait selon la revendication 1.

8. Préparation pharmaceutique destinée à la protection du corps humain ou animal contre l'endommagement oxydatif, contenant l'extrait selon la revendication 1.

9. Produit cosmétique, contenant l'extrait selon la revendication 1.

10. Procédé pour l'amélioration de la stabilité oxydative de produits alimentaires, dans lequel on ajoute à ces derniers une quantité efficace d'un extrait selon la revendication 1.

11. Utilisation d'un extrait, contenant
(a) de l'acide caféique,
(b) de l'acide sinapique ainsi qu'éventuellement
(c) des polyphénols,
étant entendu que les composants (a) et (b) sont présents en un rapport pondéral d'environ 85:15 à environ 98:2, en tant qu'antioxydant.
